# EUROPEAN PATENT APPLICATION

(11) **EP 2 937 104 A1**
(43) Date of publication of application: **28.10.2015**
(21) Application number: 13867470.0
(22) Date of filing: 24.12.2013
(51) Int. Cl.: A61L 27/14, A61L 27/54, A61L 27/58

(54) **RESORBABLE MEMBRANE FOR GUIDED BONE REGENERATION**

(30) Priority: 24.12.2012 ES 201232018
(71) Applicant: Servicio Andaluz de Salud, 41001 Sevilla (ES); Consejo Superior De Investigaciones Científicas (CSIC), 41013 Sevilla (ES); Universidad De Sevilla, 41013 Sevilla (ES); Universidad De Cádiz, 11002 Cadiz (ES)
(72) Inventor: GONZÁLEZ PADILLA, David, E-41071 Sevilla (ES); GARCÍA-PERLA GARCÍA, Alberto, E-41071 Sevilla (ES); GUTIÉRREZ PÉREZ, José Luis, E-41071 Sevilla (ES); TORRES LAGARES, Daniel, E-41013 Sevilla (ES); CASTILLO DALI, Gabriel, E-41013 Sevilla (ES); SALIDO PERACAULA, Mercedes, E-11002 Cádiz (ES); VILCHES TROYA, José, E-11002 Cádiz (ES); VILCHES PÉREZ, José Ignacio, E-11002 Cádiz (ES); TERRIZA FERNÁNDEZ, Antonia, E-41013 Sevilla (ES); BARRANCO QUERO, Ángel, E-41013 Sevilla (ES); YUBERO VALENCIA, Francisco, E-41013 Sevilla (ES); DÍAZ CUENCA, Aránzazu, E-41013 Sevilla (ES); RODRÍGUEZ GONZÁLEZ-ELIPE, Agustín, E-41013 Sevilla (ES)
(74) Representative: Hoffmann Eitle
(86) International application number: PCT/ES2013/070924
(87) International publication number: WO 2014/102431

(57) **Abstract**

The invention relates to membranes for guided bone regeneration, comprising a biodegradable polymer that has been treated with a plasma on one of the faces thereof, and on which at least one or more nanometric layers of active oxides has been deposited on one or both of the faces. The invention also relates to uses of and methods for producing said membranes and to implants based thereon.

## Description

### TECHNICAL FIELD

The present invention is encompassed within biomedicine, pharmacy and tissue engineering, and relates to a film or a membrane for guided bone regeneration, comprising a biodegradable polymer that has been treated with a plasma on one of the faces thereof, and on which one or more nanometric layers of active oxides has been deposited on one or both of the faces.

### PRIOR ART

Bone is the only tissue in the body, with the exception of embryonic tissue, that restores itself completely after an injury. However, bone tissue healing is much slower than mucosal healing, and after a surgical operation, and particularly after oral or dental surgery in which healing is desirable, it is difficult to provide conditions preventing other tissues from growing into the area in which regeneration is required. For example, in the case of extracting a substantial part of a dental root due to caries or a disease, healthy bone regeneration is desirable for replacing the extracted bone tissue. However, generally the cavity left by bone extraction is quickly filled with connective tissue and this inward growth of the connective tissue prevents bone regeneration.

The technique known as "guided bone regeneration" has been developed to solve said difficulties. In this method, a membrane is surgically inserted around the periphery of the cavity of the wound. The membrane prevents or complicates invasion of the cavity of the wound by undesired cell types and thereby allows the preferred cells to grow into the cavity, thus healing the wound.

Therefore, guided bone regeneration (GBR) is a technique the objective of which is to favor bone tissue formation with respect to connective and epithelial tissue formation in the healing process.

Two types of membrane are generally used today in guided tissue regeneration:
(1) Non-resorbable synthetic membranes which are used as barriers for GBR, and which have been approved for use in the treatment of bone defects, one of said membranes with the best results is the titanium-reinforced expanded polytetrafluoroethylene (e-PTFE) membrane, and
(2) Resorbable synthetic membranes, fundamentally those formed from copolymers of glycolic acid and polylactic acid. Resorbable membranes are classified into two types: those made with synthetic polymers and those made with natural materials. Bioabsorbable synthetic polymers are macromolecules formed by the binding of several repeating units, i.e., monomers, which are in turn formed by carbon, hydrogen, oxygen, nitrogen and, sometimes, silica and sulfur atoms. These membranes break down internally and are removed from or metabolized in the body.

Although resorbable membranes have the advantage of not requiring a second surgery, thereby reducing the morbidity of the technique, both types of membrane have serious drawbacks. Although the PTFE membrane shows suitable porosity, strength and flexibility characteristics, it is still not resorbable and therefore requires a second surgical operation for extracting same. The need for additional surgical procedures can be traumatic for the patient and can also damage the new regenerated tissue, thereby extending the treatment period. The limiting element of the second type of membrane is often the fact that decomposition products are irritants and this irritation can cause unwanted effects on the patient.

It is necessary to find a membrane providing optimal conditions for cell growth and for healing.

Various methods have been proposed for preparing resorbable membranes for guided bone regeneration applications which, when implanted in the areas of the body subjected to surgical intervention, enhance the growth of certain cells, typically osteoblasts in the case of membranes for guided bone regeneration, preventing other cells from colonizing those areas. Most of these methods are based on the use of methods in liquid phase where the active components for bone regeneration are incorporated to the membrane, generally a polymeric membrane, from aqueous solutions or the like (JP2009018086-A, KR738476-B1) or spin coating or immersion methods also from solutions (JP2009061 109(A)). Another type of approaches to this problem is based on producing complex scaffold structures where two materials disintegrate at a different speed in physiological conditions of the body, allowing the colonization of freed pores at first by cells in the body (US2011190903-A1, WO2009054609-A1; KR2009042529-A; KR946268-B1). Another proposed alternative consists of producing films or tissues with a bilayer structure, such that the outermost layer favors guided cell development and cell colonization thereof (CN20031017481 20030318, US2011060413-A1; JP2011056047-A; KR20030002224 (A)). Although in most cases these membranes are produced by means of polymers or other synthetic materials, there are also cases in which a synthetic part and another part of animal origin are incorporated in the same material (KR20030097156(A)), in this last case in the pores of the former.

The synthetic material forming the base of these membranes is usually a biodegradable polymer, use of polylactic or polyglycolic acid being common, to which there are usually added other polymers (for example, chitosan) and, most commonly, inorganic materials such as hydroxyapatite or non-crystalline varieties thereof that are not recognized as active materials for guided bone regeneration. In all these applications, the amount of active material is macroscopic and, as mentioned, it is added by means of conventional chemical synthesis methods. As demonstrated by this analysis of the state of the art, there is no commercial or protected approach in which the active ingredient, in nanometric amounts, is incorporated in thin layer form on the polymeric substrate by means of dry methods based on using plasmas or the like, as those proposed in this invention. Nor is there any commercial or protected approach in which active materials are incorporated as simple oxides to the polymeric layers serving as a base, or in which this polymeric material is subjected to plasma activation treatments to favor its decomposition in a physiological medium and therefore its reabsorption in the body.

In relation to this last point, it must be pointed out that the conventional methods for synthesizing films from degradable and biodegradable polymers such as polylactic acid or polyglycolic acid or mixtures of both are well known. However, in the materials prepared by means of these conventional methods the rate at which the degradation thereof will take place in the physiological medium cannot be readily controlled, this degradation generally being slower than that needed by the required function for a membrane for guided bone regeneration. In the scientific literature, the incorporation of pores in the structure of these layers has been postulated as a method for increasing the interaction with the medium and, as a result, the degradation rate.

It is therefore important to develop a membrane which is biodegradable, with a suitable stability and degradation rate, allowing guided bone regeneration without needing to remove the implanted material, and has nanotechnological features that can improve the bioactivity of implants, in order to promote the *in situ* conduction and osteoinduction of the implant in patients and osteoprogenitor cells, and to improve osseointegration between the implant and the surrounding bone.

### DISCLOSURE OF THE INVENTION

A first aspect of the invention relates to a membrane, hereinafter membrane of the invention, comprising a biodegradable polymer obtainable by a method which comprises exposing at least one face of a polymeric film to the effect of a plasma. In a preferred embodiment of this aspect of the invention, the plasma is made up of oxygen, argon, CO₂, N₂O or any of the mixtures thereof. More preferably, the polymer is selected from polylactic acid, polyglycolic acid or any of the mixtures thereof. In another preferred embodiment, the method for obtaining the membrane further comprises depositing one or more nanometric layers of active oxides on one or both of the faces of the polymeric film. More preferably, the active oxides are selected from the list consisting of SiO₂, TiO₂, ZnO, hydroxyapatite or derivatives, or other oxides that are neither toxic nor have side effects and favor osteoblast growth or any of the combinations thereof. In another preferred embodiment, the deposition of nanometric layers of active oxides is carried out by means of dry techniques. More preferably, the dry techniques are selected from the list consisting of physical vapor deposition (PVD), sputtering, plasma enhanced chemical vapor deposition (PECVD), pulsed laser deposition (PLD), any similar technique or any of the combinations thereof. In another preferred embodiment, the nanometric layer or layers of active oxides have a thickness ranging between 10 nm and 1 micron. Even more preferably, the nanometric layer or layers of active oxides are deposited on the face of the film or membrane not treated with plasma. In another preferred embodiment of this aspect of the invention, the time of exposing the face of the polymeric film to the effect of the plasma ranges between 10 and 40 minutes.

In another preferred embodiment, the membrane of the invention is obtained by a method which comprises:
(a) producing a resorbable film from biodegradable polymer or polymers;
(b) activating one or both of the faces of the film according to (a) by means of an oxygen plasma or a plasma made up of a mixture of gases, and preferably
(c) depositing a first layer of active material on one or both of the faces of the activated film of (b) using dry techniques that do not alter the structural integrity of the polymeric base material.

In a preferred embodiment of this aspect of the invention, the active material of step (c) is inorganic. More preferably, the method of the invention further comprises:
(d) incorporating a second or more layers in multilayer form on the first layer to achieve multifunctional membranes suitable for various clinical processes.

In another preferred embodiment, the method of the invention further comprises:
(e) depositing nanometric layers having a mixed composition by means of combining one or more simultaneous or successive deposition processes.

A second aspect of the invention relates to an implant, hereinafter implant of the invention, comprising the membrane of the invention. In a preferred embodiment of this aspect, the implant of the invention is a monolithic or articular implant. More preferably, the monolithic or articular implant is selected from sutures, staples, prostheses, screws or plates.

A third aspect of the invention relates to the use of the membrane of the invention in the elaboration of a medicinal product, or alternatively, to the membrane of the invention for use as a medicinal product.

A fourth aspect of the invention relates to the use of the membrane of the invention in the elaboration of a medicinal product for tissue regeneration, or alternatively, to the membrane of the invention for use in tissue regeneration. Preferably, it relates to the use of the membrane of the invention in the elaboration of a medicinal product for bone tissue regeneration, or alternatively, to the membrane of the invention for use in bone tissue regeneration. Even more preferably, it relates to the use of the membrane of the invention in the elaboration of a medicinal product for inducing bone tissue neoformation, or alternatively, to the membrane of the invention for inducing bone tissue neoformation. In another preferred embodiment, it relates to the use of the membrane of the invention in the elaboration of a medicinal product for repairing skeletal muscle tissue, or alternatively, to the membrane of the invention for repairing parts of skeletal muscle tissue.

A fifth aspect of the invention relates to a method for obtaining the membrane of the invention, hereinafter method of the invention, which comprises:
(a) producing a resorbable film from biodegradable polymer or polymers;
(b) activating one or both of the faces of the film according to (a) by means of an oxygen plasma or a plasma made up of a mixture of gases, and preferably
(c) depositing a first layer of active material on one or both of the faces of the activated film of (b) using dry techniques that do not alter the structural integrity of the polymeric base material.

In a preferred embodiment of this aspect of the invention, the active material of step (c) is inorganic. More preferably, the method of the invention further comprises:
(d) incorporating a second or more layers in multilayer form on the first layer to achieve multifunctional membranes suitable for various clinical processes.

In another preferred embodiment, the method of the invention further comprises:
(e) depositing nanometric layers having a mixed composition by means of combining one or more simultaneous or successive deposition processes.

### DESCRIPTION OF THE DRAWINGS

Figure 1 shows the position organization of PLGA membranes in the tegmenta of rabbits in the example with SiO₂.
Figure 2 shows O₂ plasma treatment and surface functionalization of the functionalized PLGA membranes.

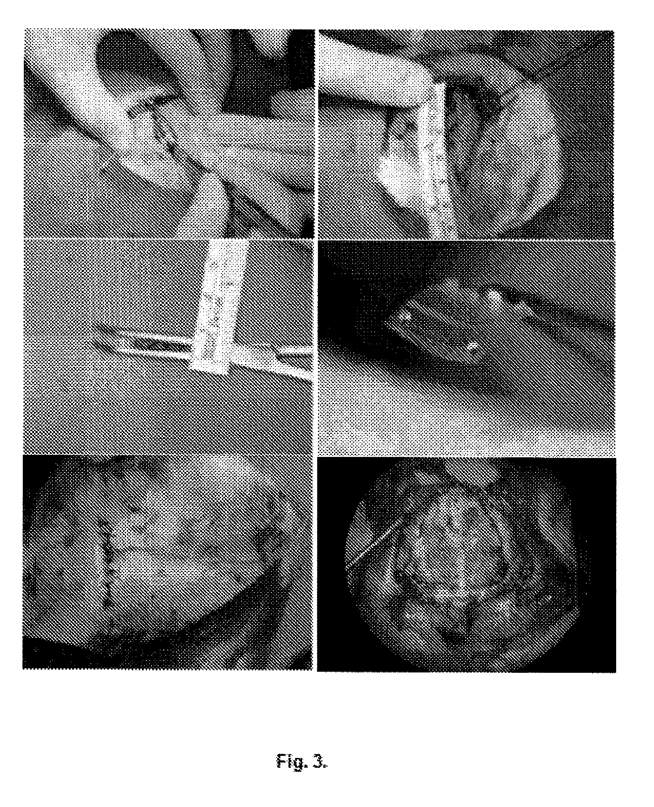

Figure 3 shows the surgical procedure. Images from the operation.

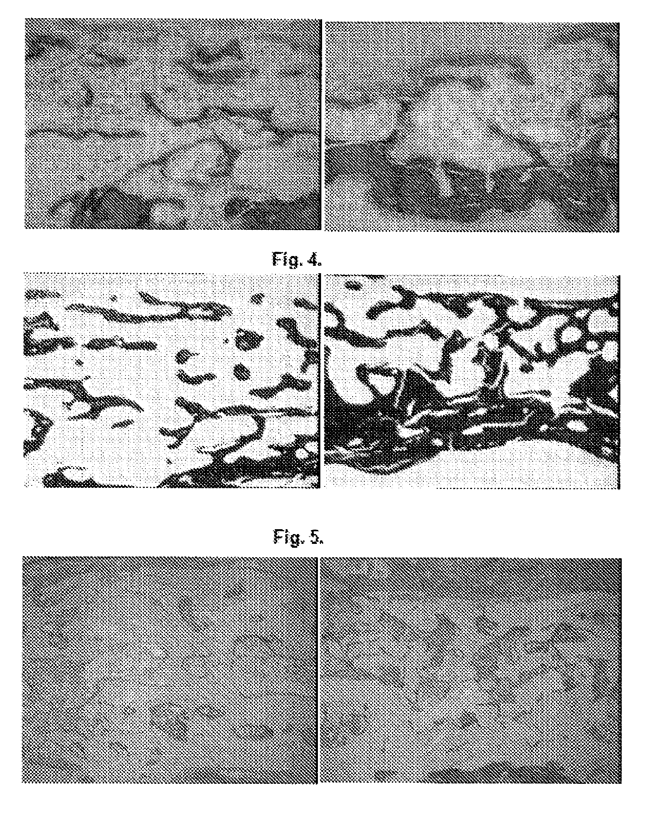

Figure 4 shows light microscopy. Stainings. Toluidine Blue (BT). Comparison of structural bone levels in the different nanocomposites with respect to control after 1 month of regeneration; (left): PLGA control. (right): SiO₂/PLGA+P-O₂. 5x objective.
Figure 5 shows light microscopy. Mineralization. Von Kossa: Comparison of mineralization levels between treatments and control one month after surgery; (left): PLGA control. (right): SiO₂/PLGA+P-O₂. 5x objective.
Figure 6 shows enzymology; resorption/apposition balance. Evidence of tartrate-resistant acid phosphatase (TRAP) of osteoclasts, resorption after 1 month of regeneration; (left): PLGA control. (right): SiO₂/PLGA+P-O₂. 5x objective.

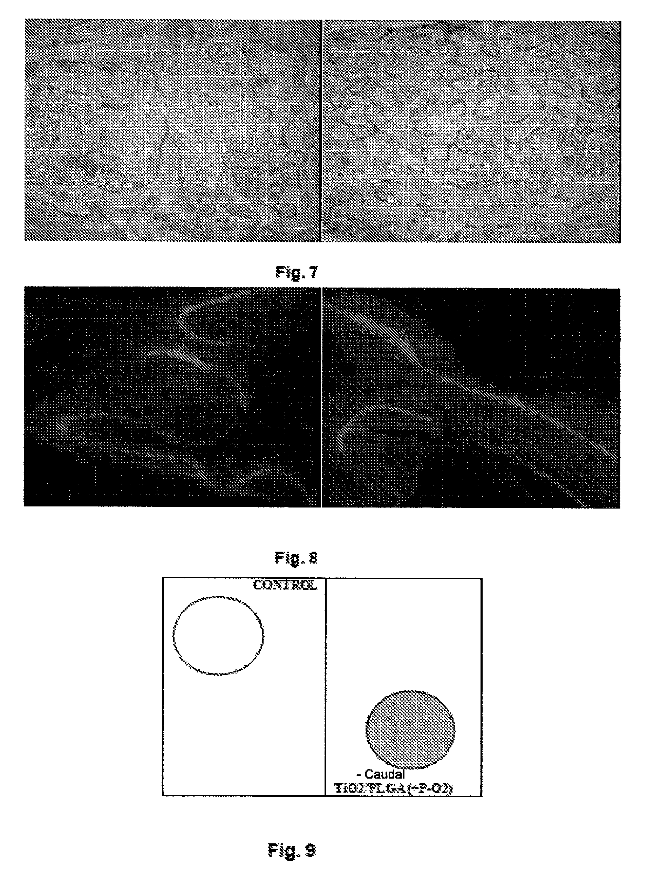

Figure 7 shows enzymology; evidence of alkaline phosphatase (ALP) of osteoblasts, apposition after 1 month of regeneration; (left): PLGA control. (right): SiO₂/PLGA+P-O₂. 5x objective.
Figure 8 shows fluorescence. Calcein study; millimeters of neoformed bone per day; (left): PLGA control. (right): SiO₂/PLGA+P-O₂. 5x objective.
Figure 9 shows the position organization of PLGA membranes in the tegmenta of rabbits in the example with TiO₂.

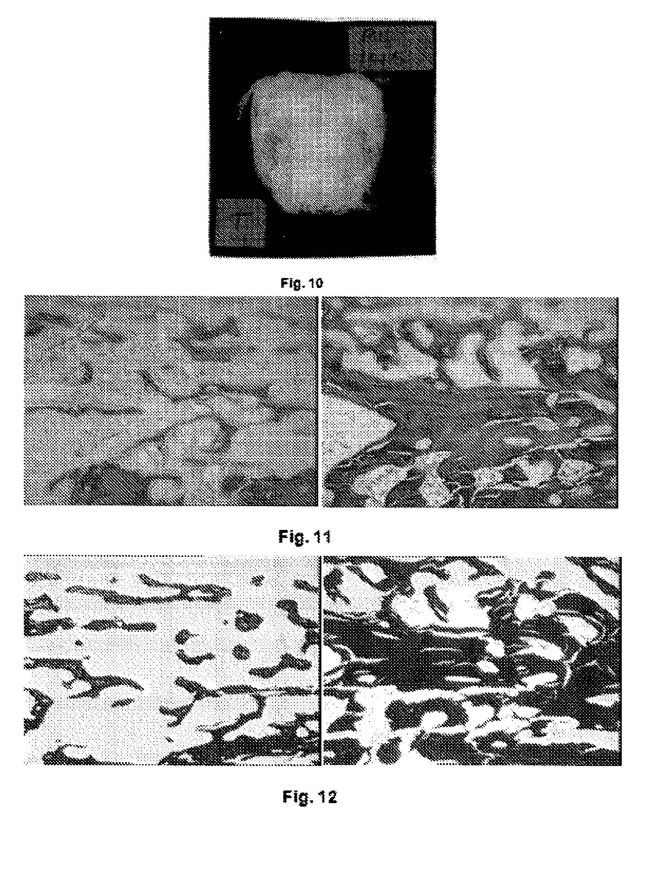

Figure 10 shows the X-ray image; TiO₂/PLGA+P-O₂. Together with control (T).
Figure 11 shows light microscopy. Stainings. Toluidine Blue (BT). Comparison of structural bone levels in the different nanocomposites with respect to control after 1 month of regeneration; (left): PLGA control. (right): TiO₂/PLGA+P-O₂. 5x objective.
Figure 12 shows light microscopy. Mineralization. Von Kossa: Comparison of mineralization levels between treatments and control one month after surgery; (left): PLGA control. (right): TiO₂/PLGA+P-O₂. 5x objective.

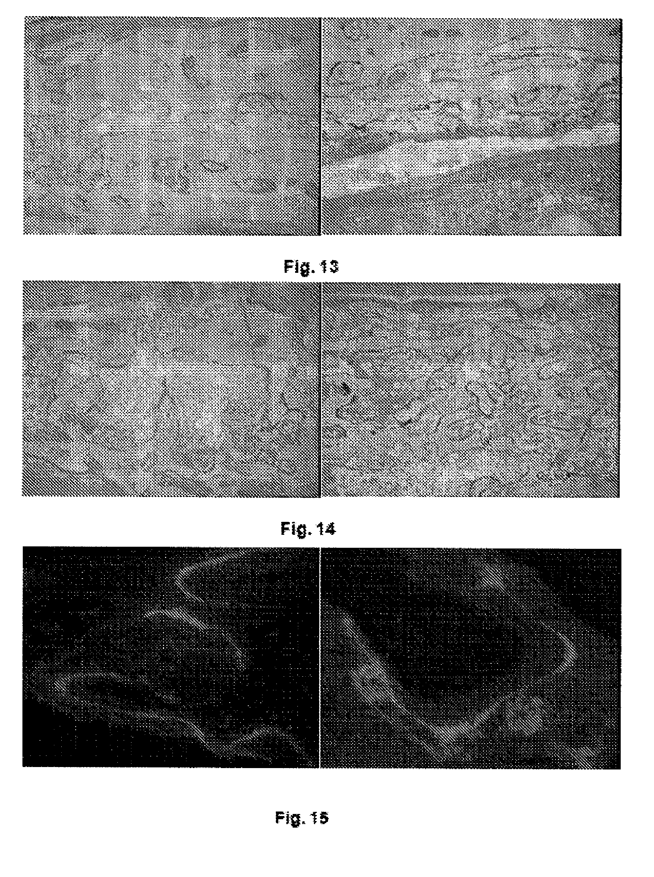

Figure 13 shows enzymology; resorption/apposition balance. Evidence of tartrate-resistant acid phosphatase (TRAP) of osteoclasts, resorption after 1 month of regeneration; (left): PLGA control. (right): TiO₂/PLGA+P-O₂. 5x objective.
Figure 14 shows enzymology; evidence of alkaline phosphatase (ALP) of osteoblasts, apposition after 1 month of regeneration; (left): PLGA control. (right): TiO₂/PLGA+P-O₂. 5x objective.
Figure 15 shows fluorescence. Calcein study; millimeters of neoformed bone per day; (left): PLGA control. (right): TiO₂/PLGA+P-O₂. 5x objective.

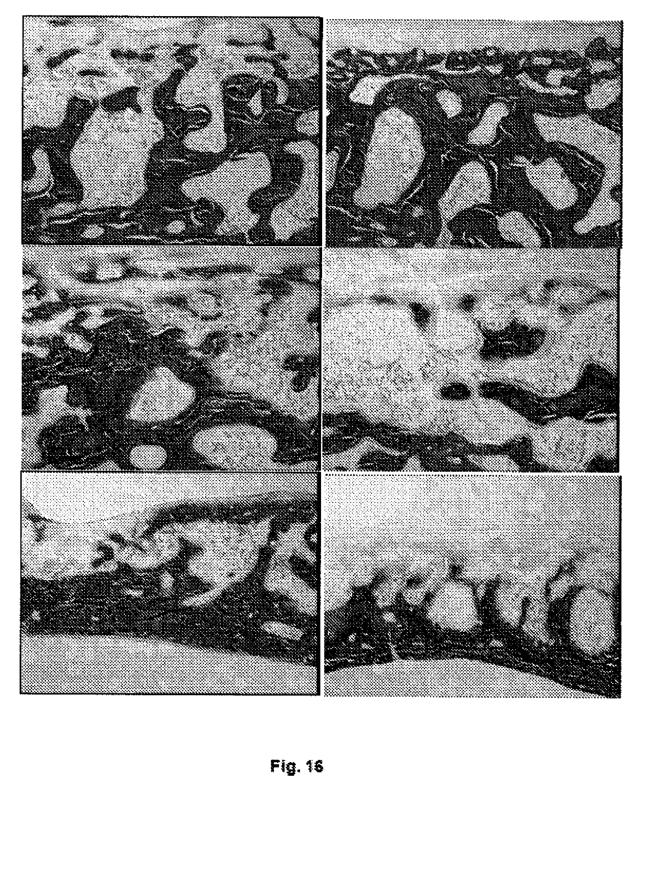

Figure 16 shows light microscopy. Stainings with Toluidine Blue (BT). Comparison using BT of the percentages of neoformed bones in the different nanocomposites after 1 month of regeneration. Images after staining with BT (1 month), 10x objective; (top left): PLGA/SiO₂. (top right): PLGA/TiO₂+O₂ plasma. (middle left): PLGA/TiO₂. (middle right): PLGA+O₂ plasma. (bottom left): PLGA/SiO₂+O₂ plasma. (bottom right): PLGA control.

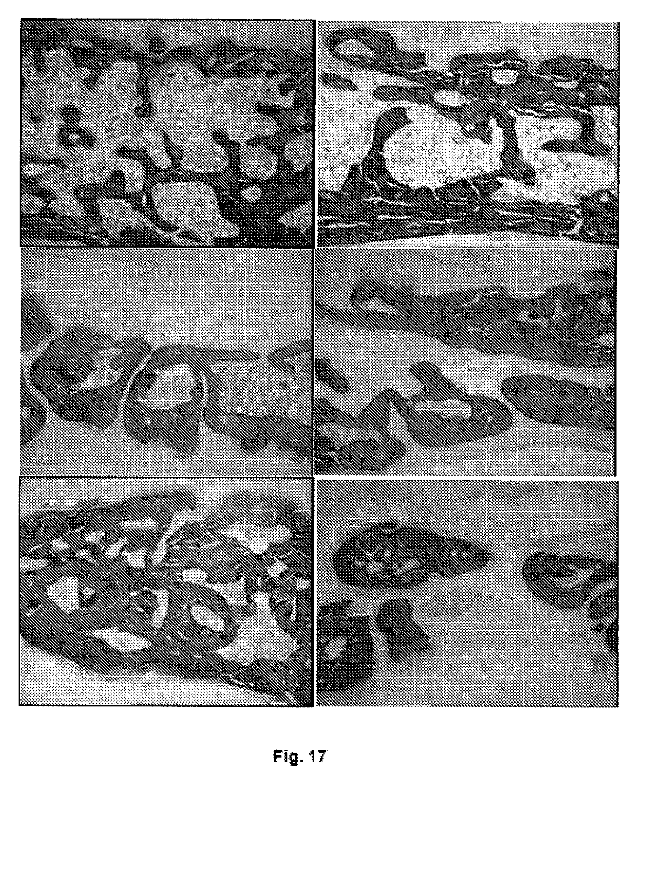

Figure 17 shows light microscopy. Stainings with Toluidine Blue (BT). Comparison using BT of the percentages of neoformed bones in the different nanocomposites after 2 months of regeneration. Images after staining with BT (2 months), 10x objective; (top left): PLGA/SiO₂. (top right): PLGA/TiO₂+O₂ plasma. (middle left): PLGA/TiO₂. (middle right): PLGA+O₂ plasma. (bottom left): PLGA/SiO₂+O₂ plasma. (bottom right): PLGA control.

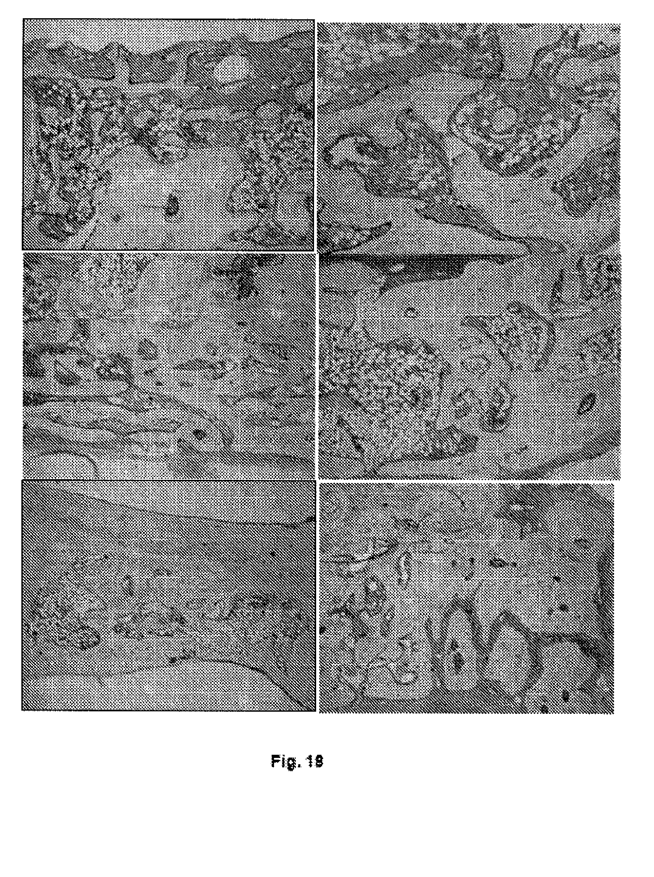

Figure 18 shows microscopy. Enzymology: evidence of tartrate-resistant acid phosphatase (TRAP) of osteoclasts after 1 month; images after enzymology with TRAP (1 month), 5x objective; (top left): PLGA/SiO₂. (top right): PLGA/TiO₂+O₂ plasma. (middle left): PLGA/TiO₂. (middle right): PLGA+O₂ plasma. (bottom left): PLGA/SiO₂+plasma 2. (bottom right): PLGA control. (contrasted with diluted Toluidine Blue).

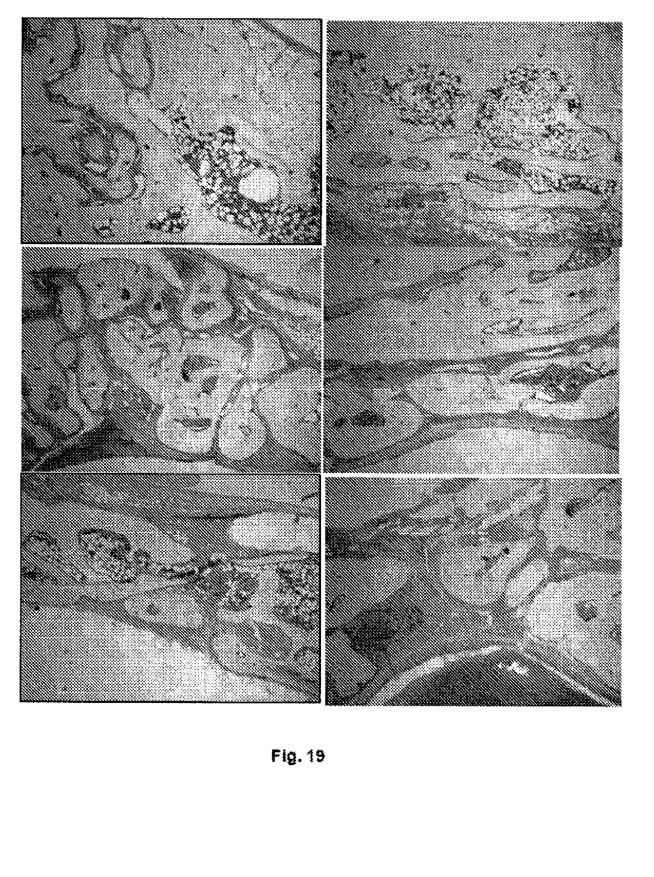

Figure 19 shows microscopy. Enzymology: evidence of tartrate-resistant acid phosphatase (TRAP) of osteoclasts after 2 months; images after enzymology with TRAP (1 month), 5x objective; (top left): PLGA/SiO₂. (top right): PLGA/TiO₂+O₂ plasma. (middle left): PLGA/TiO₂. (middle right): PLGA+O₂ plasma. (bottom left): PLGA/SiO₂+plasma 2. (bottom right): PLGA control. (contrasted with diluted Toluidine Blue).

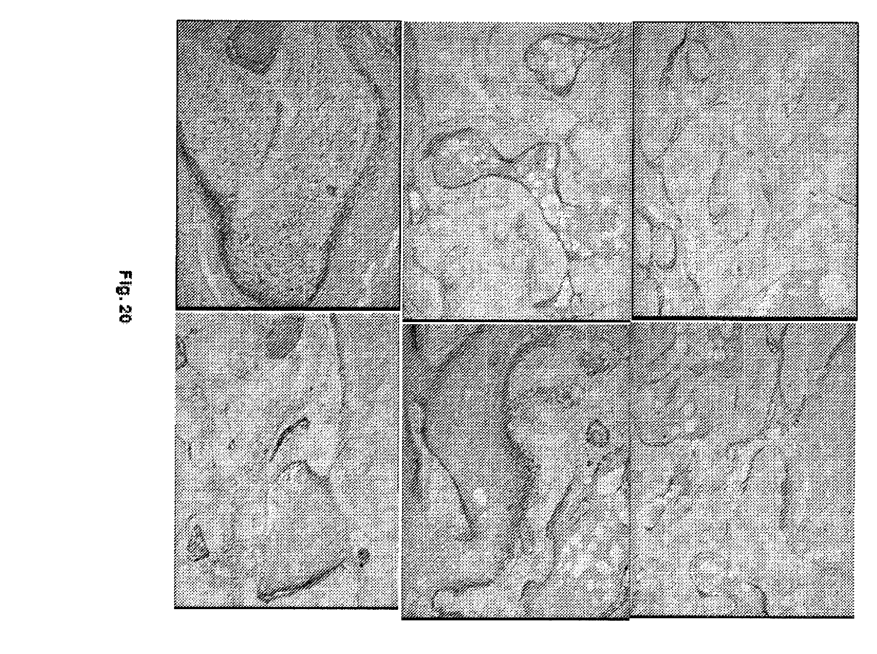

Figure 20 shows microscopy. Enzymology: evidence of alkaline phosphatase (ALP) of osteoclasts after 1 month; images after enzymology with ALP (1 month), 10x objective; (top left): PLGA/SiO₂. (top right): PLGA/TiO₂+O₂ plasma. (middle left): PLGA/TiO₂. (middle right): PLGA+O₂ plasma. (bottom left): PLGA/SiO₂+plasma 2. (bottom right): PLGA control. 20x objective.

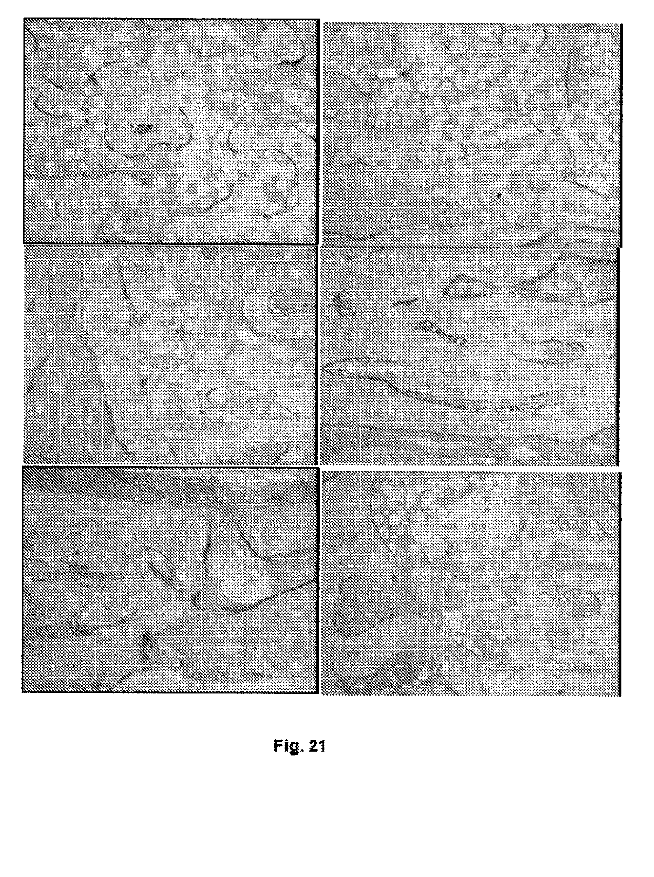

Figure 21 shows microscopy. Enzymology: evidence of alkaline phosphatase (ALP) of osteoclasts after 2 months; images after enzymology with ALP (2 months), 10x objective; (top left): PLGA/SiO₂. (top right): PLGA/TiO₂+O₂ plasma. (middle left): PLGA/TiO₂. (middle right): PLGA+O₂ plasma. (bottom left): PLGA/SiO₂+plasma 2. (bottom right): PLGA control. 20x objective.

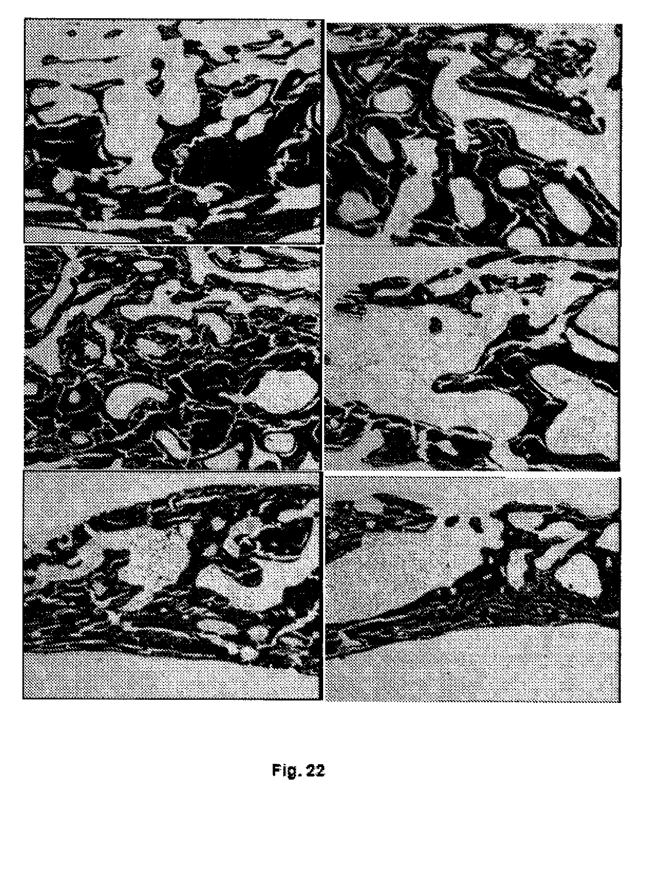

Figure 22 shows Von Kossa staining; images of the tissue after staining with Von Kossa (VK) silver nitrate after 1 month of regeneration; images after VK staining (1 month) at 10x objective; (top left): PLGA/SiO₂. (top right): PLGA/TiO₂+O₂ plasma. (middle left): PLGA/TiO₂. (middle right): PLGA+O₂ plasma. (bottom left): PLGA/SiO₂+O₂ plasma. (bottom right): PLGA control. 20x objective.

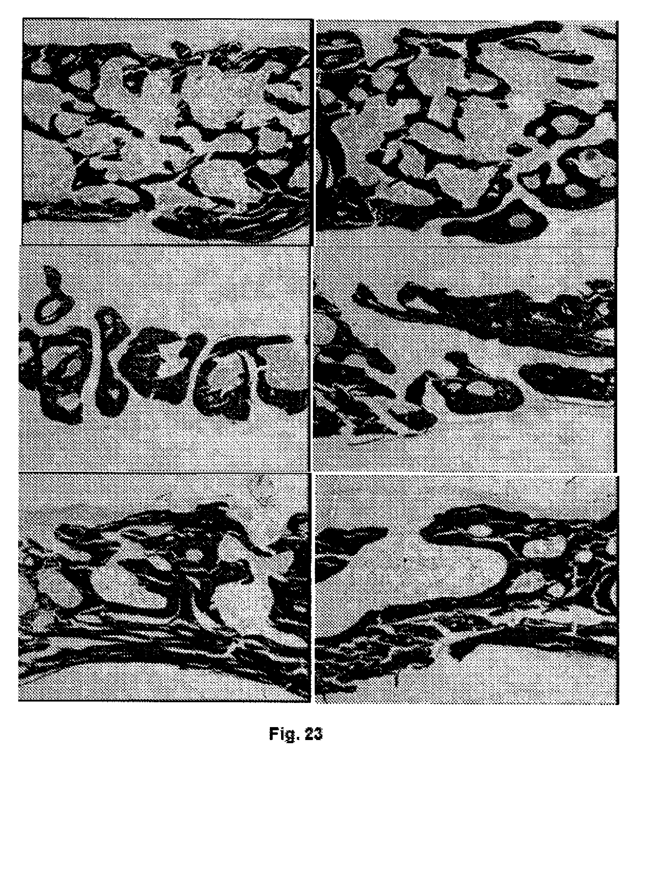

Figure 23 shows Von Kossa staining; images of the tissue after staining with Von Kossa (VK) silver nitrate after 2 months of regeneration; images after VK staining (2 months) at 10x objective; (top left): PLGA/SiO₂. (top right): PLGA/TiO₂+O₂ plasma. (middle left): PLGA/TiO₂. (middle right): PLGA+O₂ plasma. (bottom left): PLGA/SiO₂+O₂ plasma. (bottom right): PLGA control. 20x objective.

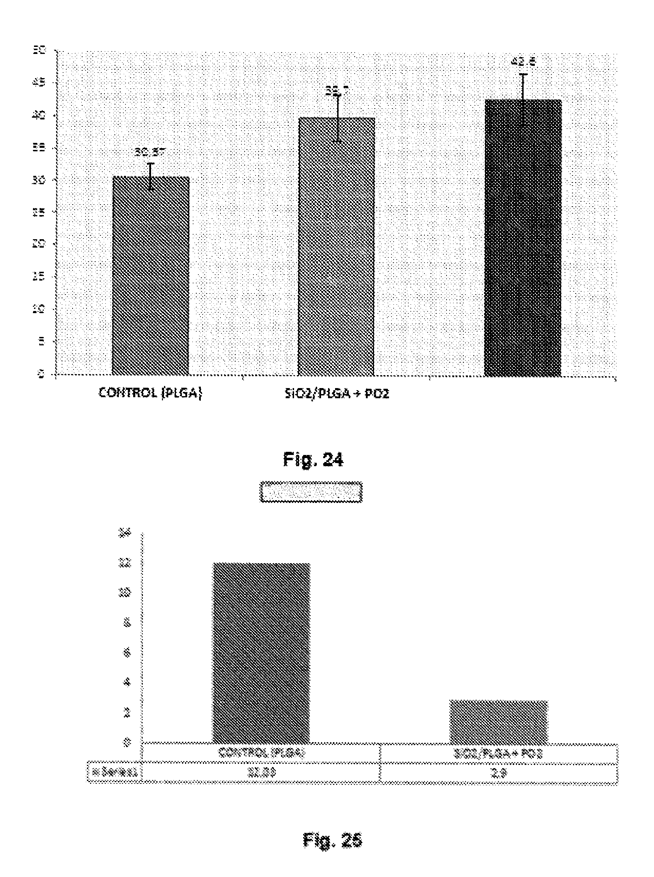

Figure 24 shows the percentage of regenerated bone after one month of regeneration. Nanocomposites of silicon oxide (SiO₂) in oxygen plasma-functionalized PLGA membranes evaluated with respect to a control (PLGA) and to the original bone (o.b.)
Figure 25 shows the differences between the original bone and the neoformed bone in each treatment with respect to control. Nanocomposites of silicon oxide (SiO₂) in oxygen plasma-functionalized PLGA membranes.

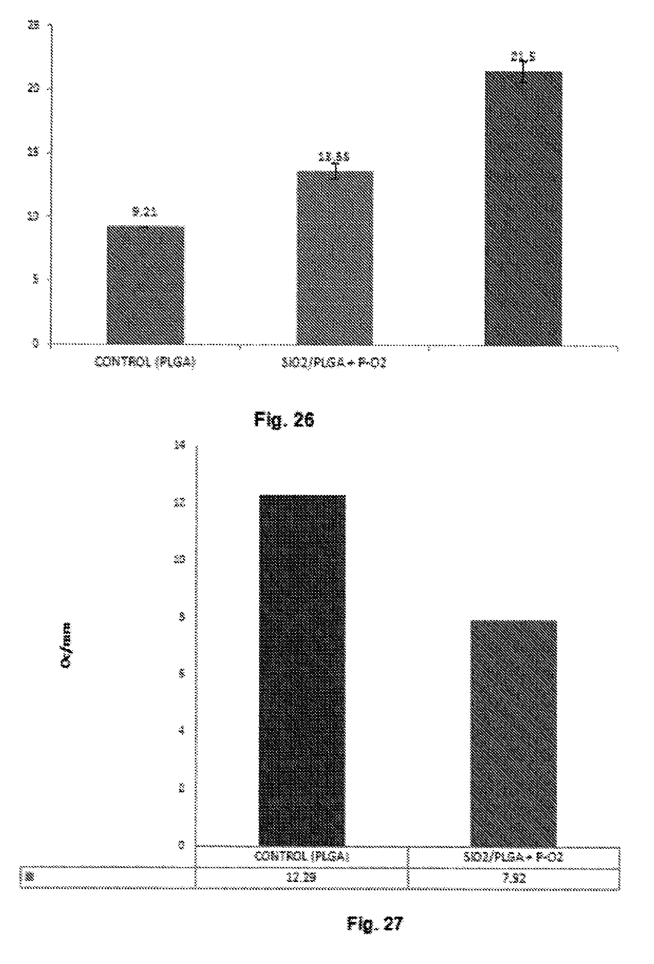

Figure 26 shows the number of osteoclasts per millimeter. Nanocomposites of silicon oxide (SiO₂) in oxygen plasma-functionalized PLGA membranes.
Figure 27 shows the differences in the resorptive level between the original bone and the neoformed bone. Nanocomposites of silicon oxide (SiO₂) in oxygen plasma-functionalized PLGA membranes.

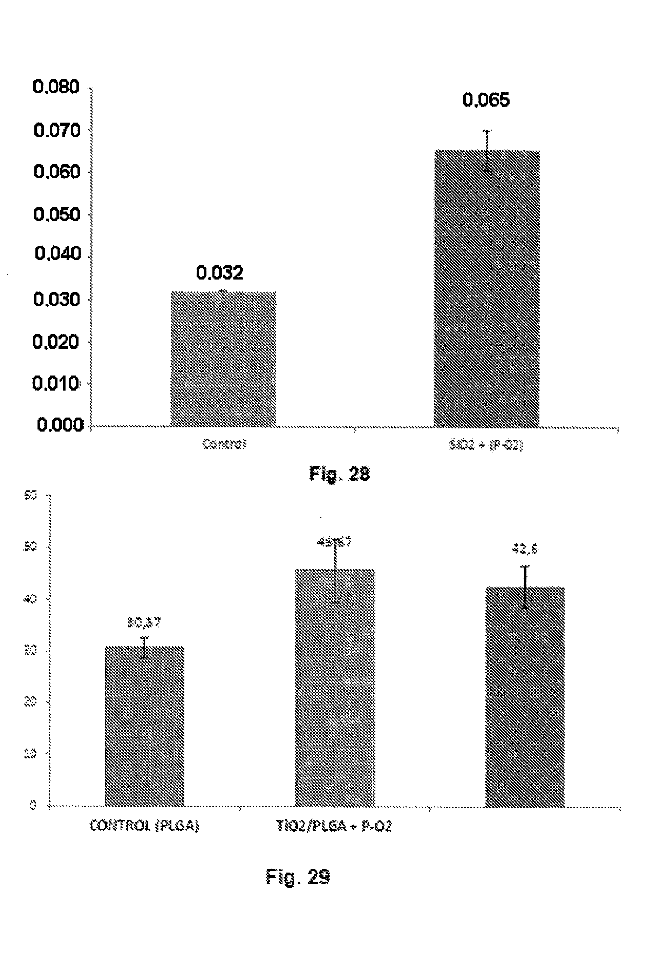

Figure 28 shows the millimeters of neoformed bone per day. Nanocomposites of silicon oxide (SiO₂) in oxygen plasma-functionalized PLGA membranes.
Figure 29 shows the percentage of regenerated bone after one month of regeneration. Nanocomposites of titanium oxide (TiO₂) in oxygen plasma-functionalized PLGA membranes.

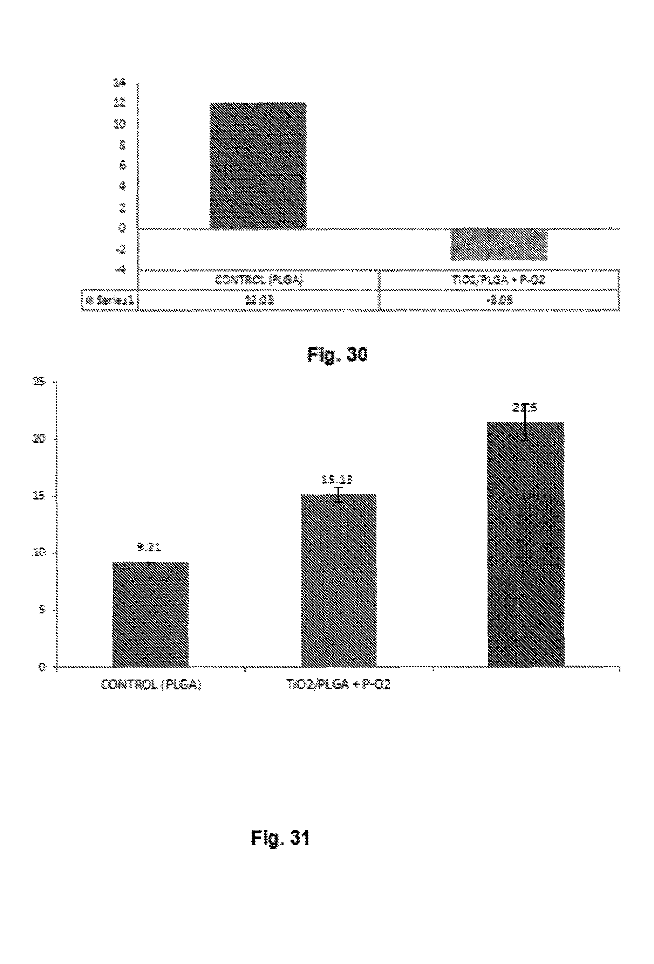

Figure 30 shows the differences between the original bone and the neoformed bone in each treatment with respect to control. Nanocomposites of titanium oxide (TiO₂) in oxygen plasma-functionalized PLGA membranes.
Figure 31 shows the number of osteoclasts per millimeter. Nanocomposites of titanium oxide (TiO₂) in oxygen plasma-functionalized PLGA membranes.

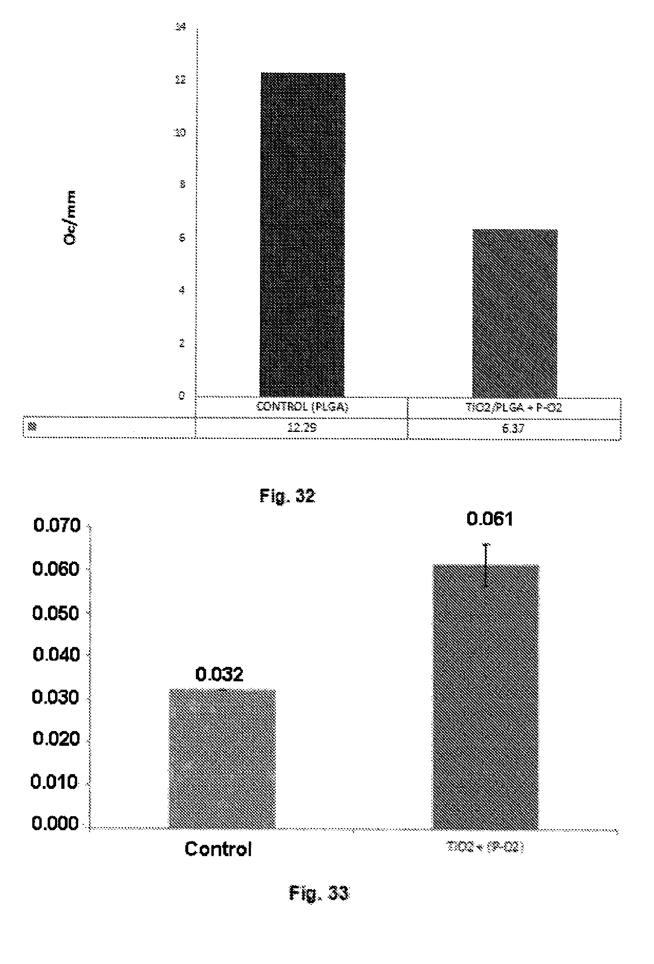

Figure 32 shows the differences between the resorptive level of the original bone and the neoformed bone. Nanocomposites of titanium oxide (TiO₂) in oxygen plasma-functionalized PLGA membranes.
Figure 33 shows the millimeters of neoformed bone per millimeter. Nanocomposites of titanium oxide (TiO₂) in oxygen plasma-functionalized PLGA membranes.

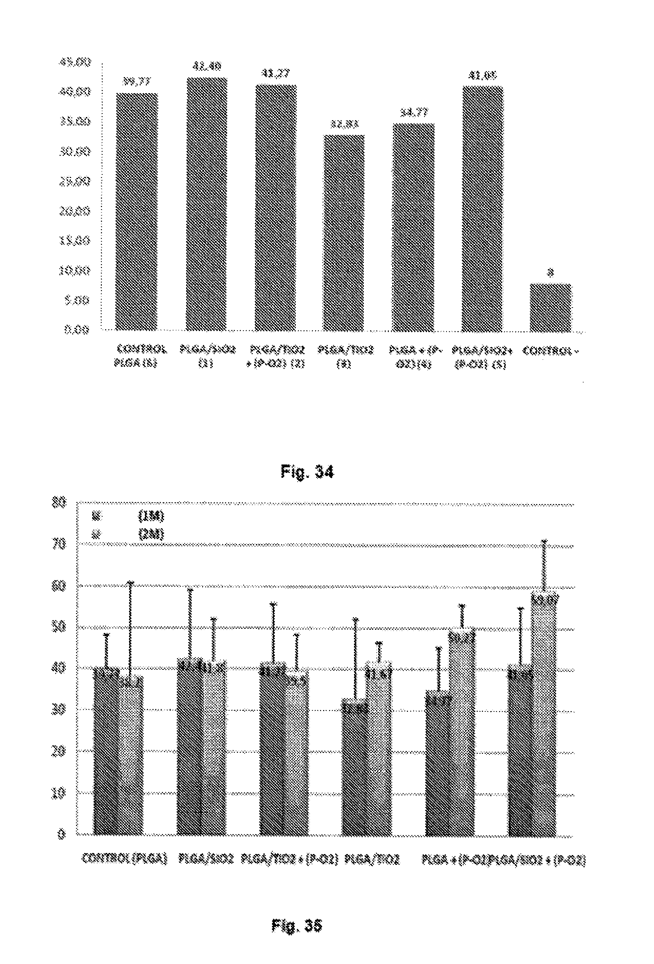

Figure 34 shows the comparison of the percentages of neoformed bones after 1 month with respect to the control without membrane.
Figure 35 shows the comparison of the mean percentage of neoformed bone in 1 and 2 months.

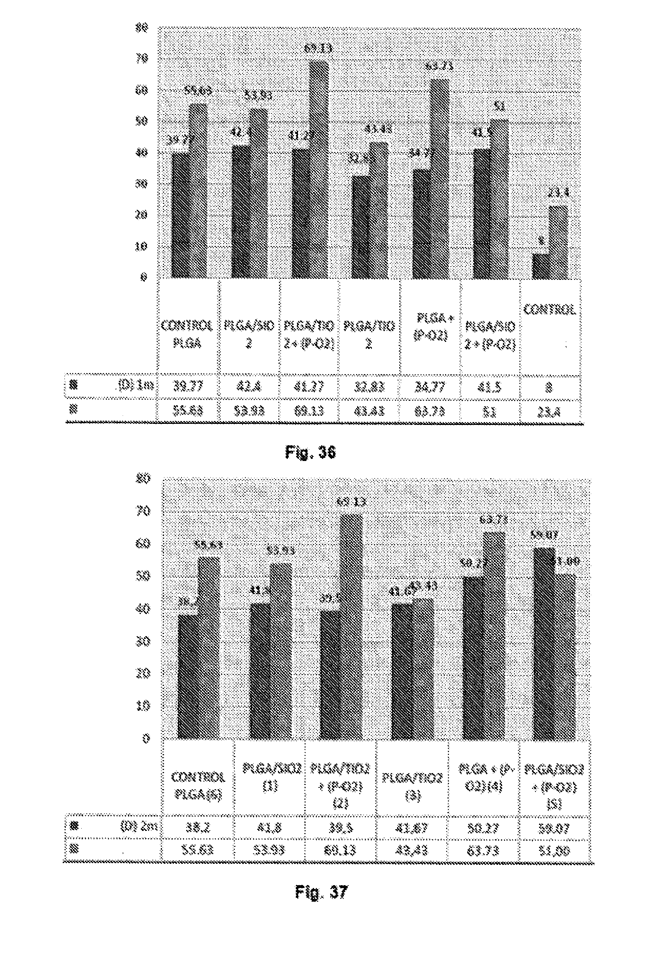

Figure 36 shows the comparison of the percentage of neoformed bone after 1 month with respect to the original bone.
Figure 37 shows the comparison of the percentage of neoformed bone after 2 months with respect to the original bone.

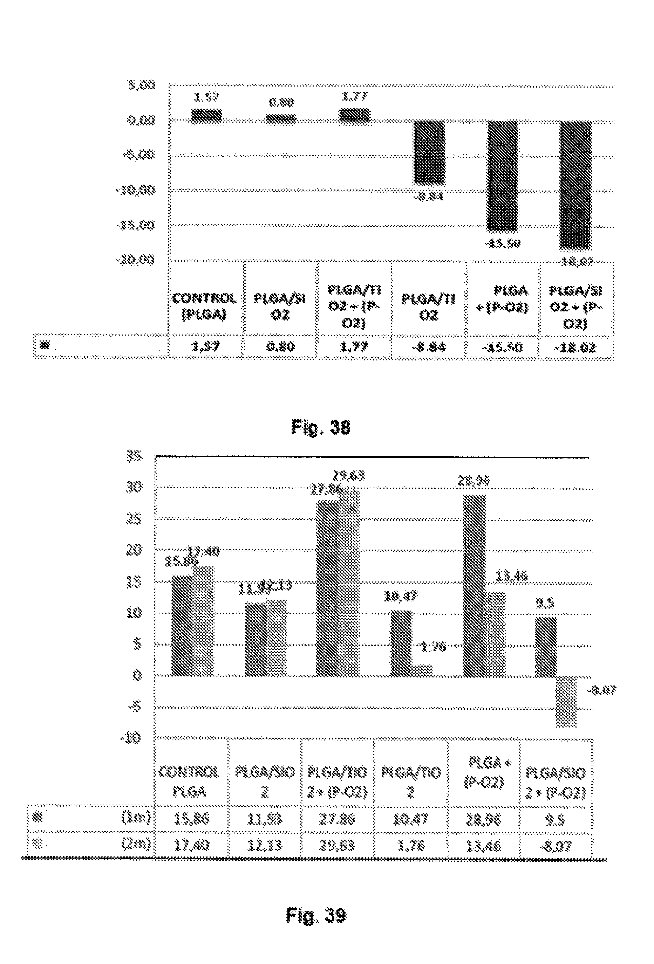

Figure 38 shows the comparison of the differences between the percentage of neoformed bone in 1 and 2 months.
Figure 39 shows the comparison of the differences between the percentage of neoformed bone with respect to the pre-existing original bone in 1 and 2 months after regeneration.

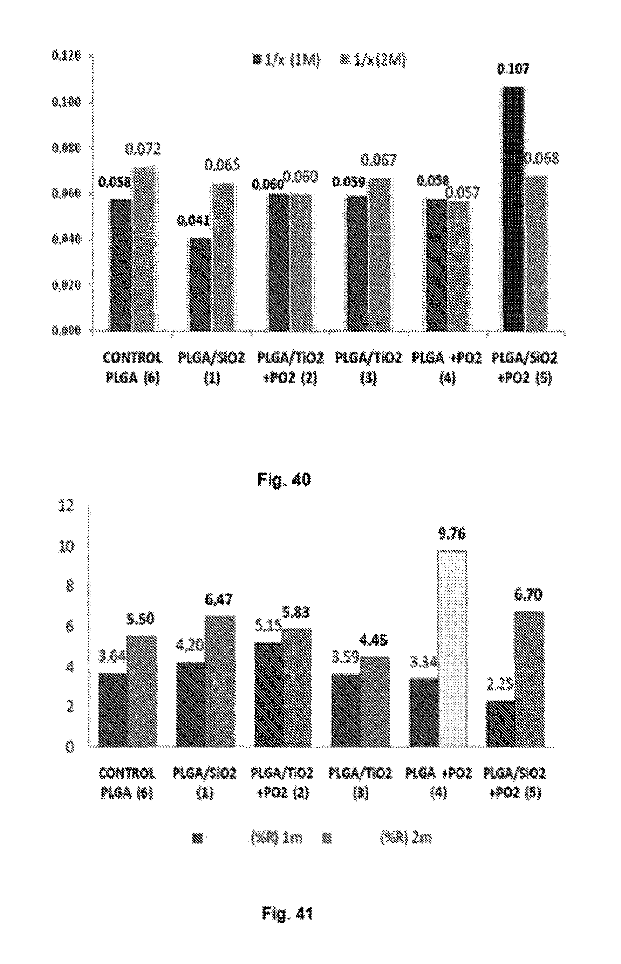

Figure 40 shows the comparison of the inverse of the means of the mineralized bone lengths in 1 and 2 months for each type of membrane.
Figure 41 shows the comparison of the resorptive percentage in each type of membrane after 1 and 2 months.

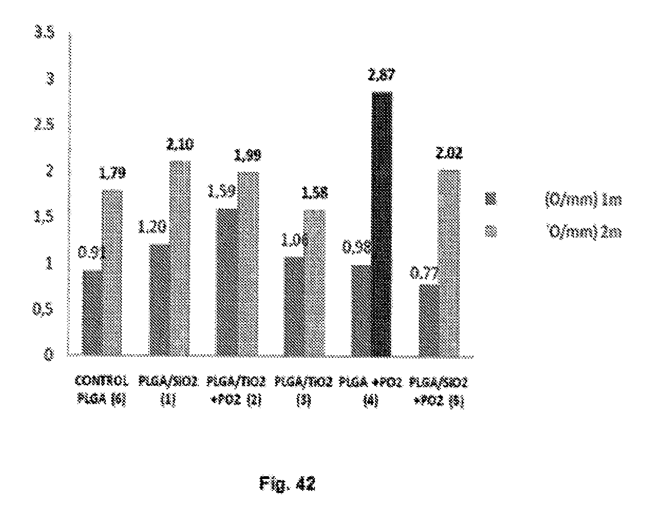

Figure 42 shows the comparison of the number of osteoclasts per millimeter of each type of membrane after 1 and 2 months.

### DETAILED DISCLOSURE OF THE INVENTION

The authors of the present invention have developed a resorbable polymeric membrane or film that is biodegradable (undergoes hydrolysis when it comes into contact with the physiological medium), sufficiently stable, with great design flexibility, and allows adapting the composition and structure thereof to specific needs. The authors of the invention control the polymer degradation rate by means of plasma treatments. The improvements in the properties of the membranes thus constructed are not only limited to mechanical properties, this not being the main objective of the invention either. The bioactivity of the membrane is improved, improving and modifying cell and tissue response.

### MEMBRANE OF THE INVENTION

Although well documented, the biodegradability of polymeric membranes or films in a real *"in vivo"* physiological medium or their *"in vitro"* imitation is limited, sometimes needing times much longer than the times required in clinical practice. To enable controlling and reducing these degradation times, this invention has developed a method which consists of activating one or both of the faces of the synthesized films by exposing same to an oxygen plasma or a plasma made up of another active gas generated in a conventional plasma reactor (argon, oxygen and argon mixtures, CO₂, N₂O or mixtures thereof, etc., this invention including all the possibilities capable of generating active species producing physical and/or chemical surface erosion effects on the surface of the polymer). The inventors thereby obtain a resorbable membrane with a controlled degradation rate in the physiological medium.

### Polymer

Therefore, a first aspect relates to a membrane, hereinafter membrane of the invention, comprising a biodegradable polymer obtainable by a method which comprises exposing at least one face of a polymeric film to the effect of a plasma. Said plasma can be an oxygen plasma or a plasma made up of any other active gas generated in a conventional plasma reactor, this invention including all the possibilities capable of generating active species producing physical and/or chemical surface erosion effects on the surface of the polymer. In a preferred embodiment of this aspect of the invention, the plasma is made up of oxygen, argon, CO₂, N₂O or any of the mixtures thereof. According to the required function and the reabsorption time required for same, these membranes or films can have a variable thickness comprised between a few hundred microns of thickness to half a millimeter or more, the manageability thereof being another criterion used to define this parameter.

Layers of polymers such as polylactic acid, polyglycolic acid or mixtures of both prepared by means of conventional methods are used as polymeric films acting as substrates and element for separating the areas of the body subjected to surgical intervention. Therefore, in another preferred embodiment of this aspect of the invention the polymer is selected from polylactic acid, polyglycolic acid or any of the mixtures thereof.

By means of the claimed plasma technology, the polymeric film degradation rate can be controlled by changing either the polymer activation treatment time, favoring the degradation for longer times, or the plasma conditions. In this case, the effects are maximized by increasing the concentration of active species in the plasma by means of all the adjustable parameters characteristic of this technology (plasma power, characteristics of the electromagnetic radiation used for activating the plasma, pressure of the gases, flow thereof, etc.).

### Nanometric layer of active oxides

The present invention proposes the activation of one or both of the faces of the membranes with respect to the colonization thereof with osteoblasts or other cells by means of incorporating nanometric layers of active oxides on the surface of the polymeric film.

Therefore, in another preferred embodiment the membrane of the invention is obtained by a method which further comprises depositing one or more nanometric layers of active oxides on one or both of the faces of the polymeric film. In another more preferred embodiment, the active oxides are selected from the list consisting of SiO₂, TiO₂, ZnO, hydroxyapatite or derivatives or any of the combinations thereof, or other oxides that are neither toxic nor have side effects and favor osteoblast growth.

To prevent membrane alteration, a critical element of the present invention is that the incorporation of that active material, typically crystalline or amorphous simple oxides such as SiO₂, TiO₂ or other similar oxides, or compounds such as hydroxyapatite or the like, is carried out by means of a dry method preserving the integrity of the base film. The thickness of this active layer can be variable between tens to hundreds of nanometers or even several microns, the fact that the active layer being able to separate from the polymeric substrate due to stress accumulation being at times a restriction for the case of thicker layers. In the case of a thinner layer, it can be continuous and conformal with the intrinsic roughness of the substrate or it may not form a continuous layer but rather develop in the form of nanometric islets without continuity throughout the surface.

Both the activation process for controlling the polymer degradation rate and the incorporation of active nanometric layers on the surface is carried out by means of plasma-based dry techniques in the complete absence of a liquid precursor medium. The fundamental advantage of dry deposition using plasmas or related techniques (such as for example, but without limitation, magnetron sputtering) is that it allows depositing these materials at a temperature close to room temperature, which allows obtaining amorphous phases or low crystallinity phase and in turn eliminating the risk of the polymer degrading thermally. In addition, these techniques allow incorporating the aforementioned oxides and compounds on the polymer in a controlled manner. Likewise, they allow obtaining very homogenous layers having controlled thickness, in turn assuring great properties in terms of adhesion thereof to the polymeric base film.

Therefore, in a preferred embodiment the incorporation of active nanometric layers on the surface is carried out by means of plasma-based dry techniques or the like. In another preferred embodiment, the dry techniques are selected from the list consisting of physical vapor deposition (PVD), sputtering, plasma enhanced chemical vapor deposition (PECVD), pulsed laser deposition (PLD), any similar technique or any of the combinations thereof. For any of these methods, a critical element is that there are no processes of heating the polymeric substrate and that the material of the active nanometric layer is provided without structurally modifying the base film.

The thickness of this active layer can be variable between tens to hundreds of nanometers or even several microns, the fact that the active layer being able to separate from the polymeric substrate due to stress accumulation being at times a restriction for the case of thicker layers. In the case of thinner layers, it can be continuous and conformal with the intrinsic roughness of the substrate or it may not form a continuous layer but rather develop in the form of nanometric islets without continuity throughout the surface. Therefore, in another preferred embodiment the nanometric layer or layers have a thickness ranging between 10 nm and 1 micron. More preferably, it ranges between 30 and 200 nm, and even more preferably, it ranges between 50 and 100 nm. Therefore, for example, the thickness could be, but without limitation, 10 nm, 15 nm, 20 nm, 25 nm, 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 200 nm, 300 nm, 400 nm, 500 nm, 600 nm, 700 nm, 800 nm, 900 nm, or 1 micron. For conventional layer deposition systems, these thicknesses can be achieved, for example, but without limitation, between 3 and 20 minutes, assuming deposition rates of about 10 nm/min⁻¹.

In another preferred embodiment, the face of the polymeric film is exposed to the effect of a plasma for a time ranging between 2 and 60 minutes, more preferably between 5 and 50 minutes, even more preferably between 8 and 45 minutes, and even much more preferably between 10 and 40 minutes.

The membrane of the invention can further comprise other active ingredients such as growth factors, bone morphogenetic proteins, and in general, any simple or complex molecule which has beneficial effects, particularly, but without limitation, on bone growth, and which is not toxic for the body.

In another preferred embodiment, the membrane of the invention is obtained by a method which comprises:
(a) producing a resorbable film from biodegradable polymer or polymers;
(b) activating one or both of the faces of the film according to (a) by means of an oxygen plasma or a plasma made up of a mixture of gases, and preferably
(c) depositing a first layer of active material on one or both of the faces of the activated film of (b) using dry techniques that do not alter the structural integrity of the polymeric base material.

In a preferred embodiment of this aspect of the invention, the active material of step (c) is inorganic. More preferably, the method of the invention further comprises:
(d) incorporating a second or more layers in multilayer form on the first layer to achieve multifunctional membranes suitable for various clinical processes.

In another preferred embodiment, the method of the invention further comprises:
(e) depositing nanometric layers having a mixed composition by means of combining one or more simultaneous or successive deposition processes.

Another aspect of the invention relates to an implant comprising the membrane of the invention. The implant can take any shape that is suited to the bone region to be regenerated. In a preferred embodiment of this aspect of the invention, the implant is a monolithic or articular implant which is selected from the list consisting of sutures, staples, prostheses, screws or plates.

### USES OF THE MEMBRANE OF THE INVENTION

The membrane of the authors of the invention is capable of regenerating bone tissue by inducing neoformation of said tissue. Therefore, another aspect of the invention relates to the use of the membrane of the invention in the elaboration of a medicinal product, or alternatively, to the membrane of the invention for use as a medicinal product.

Another aspect of the invention relates to the use of the membrane of the invention in the elaboration of a medicinal product for tissue regeneration, or alternatively, to the membrane of the invention for use in tissue regeneration.

Another aspect of the invention relates to the use of the membrane of the invention in the elaboration of a medicinal product for bone tissue regeneration, or alternatively, to the membrane of the invention for use in bone tissue regeneration.

Another aspect of the invention relates to the use of the membrane of the invention in the elaboration of a medicinal product for inducing bone tissue neoformation, or alternatively, to the membrane of the invention for inducing bone tissue neoformation.

Another aspect of the invention relates to the use of the membrane of the invention in the elaboration of a medicinal product for repairing parts of skeletal muscle tissue, or alternatively, to the membrane of the invention for repairing parts of skeletal muscle tissue.

As it is used herein, the term "medicinal product" refers to any substance used for preventing, diagnosing, relieving, treating or curing diseases in humans and animals. Therefore, in the context of the present invention, the medicinal product is used for the treatment of a bone disease.

### METHOD FOR OBTAINING THE MEMBRANE OF THE INVENTION

Another aspect relates to a method for obtaining the membrane of the invention, hereinafter method of the invention, which comprises exposing at least one face of a polymeric film to the effect of a plasma.

The new process, an object of the present invention, is based on applying an activation process for activating the surface of a biodegradable polymeric film by means of oxygen plasma or plasma made up of a mixture of gases generating active species capable of producing physical and/or chemical etching of the surface of the polymeric film. These species are applied on the polymeric film supported on a substrate. Among other effects, this treatment causes a significant increase in the surface roughness of the film, as well as the controlled alteration of the chemical composition of the first surface layers with the breakdown of bonds between polymeric chains and the incorporation of a high concentration of functional groups containing oxygen that are attacked preferably in aqueous or physiological media. In the case of external treatments, significant internal porosity connected with the exterior making the entry of external agents easier may be generated. These effects favor polymer degradation, a process which can be controlled by changing plasma treatment intensity.

In a preferred embodiment of this aspect, the plasma is made up of oxygen, argon, CO₂, N₂O or any of the mixtures thereof. In another preferred embodiment, the face of the polymeric film is exposed to the effect of a plasma for a time ranging between 2 and 60 minutes, more preferably between 5 and 50 minutes, even more preferably between 8 and 45 minutes, and even much more preferably between 10 and 40 minutes. In another preferred embodiment, the polymer is selected from polylactic acid, polyglycolic acid or any of the mixtures thereof.

Such activation can be carried out in reactors at a low pressure or at atmospheric pressure, in radiofrequency-activated plasma reactors, microwaves or of high voltage, the present invention covering all the possibilities of cold plasmas with the only restriction that the process must occur at room temperature and alter only the surface part of the film exposed to the plasma. An example of an application of this activation process consists of using a radiofrequency-operated plasma reactor having parallel plates for generating an oxygen plasma at a working pressure comprised between 1x10⁻⁴ and 2x10⁻⁴ bars and a power comprised between 10 and 20 W. By means of the claimed plasma technology, the polymeric film degradation rate can be controlled by changing either the polymer activation treatment time, favoring the degradation for longer times, or the plasma conditions. In this case, the effects are maximized by increasing the concentration of active species in the plasma by means of all the adjustable parameters characteristic of this technology (plasma power, characteristics of the electromagnetic radiation used for activating the plasma, pressure of the gases, flow thereof, etc.). In the second step of the process for producing the claimed membrane for guided bone regeneration, a continuous layer (n) or a fragmented layer (n) having controlled thickness between a few tens of nanometers and the range of microns, consisting of a layer of an inorganic material which activates cell growth proliferation on the surface thereof, is deposited either on the surface not activated by the plasma or on the activated surface. Generally, but without limitation, a face of the polymeric film is preferably treated with plasma and the active material is deposited on the other face, however, other options are possible. The membrane thus prepared is implanted in the area of the body to be regenerated by placing the inorganic part towards the area where the bone tissue must be regenerated.

In another preferred embodiment, the method of the invention further comprises depositing one or more nanometric layers of active oxides on one or both of the faces of the polymeric film. More preferably, the active oxides are selected from the list consisting of SiO₂, TiO₂, ZnO, hydroxyapatite or derivatives, or other oxides that are neither toxic nor have side effects and favor osteoblast growth or any of the combinations thereof. Even more preferably, the incorporation of active nanometric layers on the surface is carried out by means of dry techniques. Even much more preferably, the dry techniques are selected from the list consisting of physical vapor deposition (PVD), sputtering, plasma enhanced chemical vapor deposition (PECVD), pulsed laser deposition (PLD), any similar technique or any of the combinations thereof.

Many of these methods exist in small-scale laboratory varieties and in industrial scale modality in large reactors or in others where the polymeric film can be treated according to a roll-to-roll approach.

It is important to point out the possibility of the method of the invention for producing nanometric layers having a mixed composition by means of the combination of several simultaneous or successive deposition processes.

The process is compatible with the use of one or more inorganic precursors in the case of PECVD processes or of "blanks" in the case of PVD, which therefore allows incorporating one or more inorganic compounds sequentially or simultaneously. If the process is of the PECVD type, the volatile metalorganic precursors decompose upon interacting with the plasma. Since they are generally (although not always) formed by carbon, hydrogen or other elements, due to reaction with the oxygen species of the plasma, they give rise to volatile compounds (CO₂, H₂O, etc.) which are removed from the reaction chamber by the same pumping system that is used for keeping the pressure of the gas or mixture of gases used for maintaining plasma discharge constant within the chamber.

Simultaneously, as it is oxidized by the species of the plasma, the metal element present in the compound used is incorporated to the thin layer of oxide that is being formed. The inorganic layer deposition rate can be varied by controlling the partial pressure of the metalorganic compound(s) and the plasma conditions.

The following must be mentioned as advantages of the process object of the invention:
- The base polymer degradation rate can be controlled by simply adjusting the plasma treatment time;
- A wide range of inorganic layers can be synthesized by searching for a given specificity with respect to the growth of certain cells (for example, SiO₂, TiO₂, ZnO, hydroxyapatite...). To that end, changing the blank of the material to be evaporated in PVD techniques or the volatile organometallic precursor in PECVD techniques is sufficient;
- There is a wide range of commercially available metalorganic complexes or blanks for evaporation;
- It can be easily implemented in already existing PVD or PECVD equipment;
- Thermal evaporation techniques, magnetron sputtering techniques, PECVD techniques or the like are cost effective and widely implemented at the industrial level;
- The independent control of several sources for the material of the active layer allows adjusting the multilayer composition and/or structure of the resulting material;
- Since it is a process performed at room temperature, it is suitable for heat-sensitive polymeric substrates, not being restricted to resorbable polymeric substrates the first object of this invention.

Both the composition and the thickness of the deposited layer of active material can be controlled by means of the following parameters:
- Type of precursor or material of the blank used;
- Deposition process time; and
- Physical arrangement or architecture of the reactor for carrying out the method.

Generally, the claimed production method is developed in conventional vacuum equipment for deposition by means of PVD techniques, PECVD techniques or the like.

Additionally, the device or devices used can also comprise means for controlling the deposition process *"in situ",* such as, for example, quartz balances for monitoring the deposited thicknesses, located close to the sample holder and with a variable geometry. Likewise, for optimizing the process it is suitable to have systems that allow mobility under vacuum conditions of the sample holder and the deposition sources.

Throughout the description and claims the word "comprises" and variants thereof do not intend to exclude other technical features, supplements, components or steps. For persons skilled in the art, other objects, advantages and features of the invention will be understood in part from the description and in part from the practice of the invention. The following examples and drawings are provided by way of illustration and are not meant to limit the present invention.

### EXAMPLES OF THE INVENTION

The invention will be illustrated below by means of assays conducted by the inventors, which clearly shows the specificity and effectiveness of the membrane of the invention in bone tissue regeneration, and particularly for guided bone regeneration.

The examples evaluate the potential of the new membrane developed with polyglycolic acid (PLGA) for bone regeneration by modifying the surface thereof on the nanometric scale (oxygen plasma, PO₂) and incorporating layers of TiO₂ or SiO₂.

To that end, three New Zealand rabbits were studied, producing defects (5 mm) on their skull. A membrane of each type (PLGA-PO₂/PLGA-SiO₂/PLGA-TiO₂/PLGA-SiO₂/PO₂/PLGA-TiO₂/PO₂/PLGA) was placed in each defect. The animals were sacrificed after two months. The following data was collected: percentage of neoformed bone, mineralized bone length, percentage of bone reabsorption and osteoclasts per millimeter.

The histological sections showed a layer of bone in a fairly advanced phase of formation without signs of necrosis. In relation to the percentage of neoformed bone, the best values were obtained with the PLGA/SiO₂/PO₂ membrane (59.07% ± 12.15). All the study groups showed values greater than the PLGA group (38.20% ± 22.67). The data shows a trend indicating that the functionalization of the PLGA membrane improves bone regeneration when it is applied to skull defects in an animal model. By comparing the functionalization of TiO₂ and SiO₂, as well as PO₂/TiO₂ and PO₂/SiO₂, of PLGA membranes, the functionalization of silicon seem to provide more promising results than titanium.

### Example 1. In vivo comparative evaluation of the nanocomposites of silicon oxide (SiO₂) in oxygen plasma-functionalized PLGA membranes, by means of GBG

The *in vivo* functional differences between treatment with nanocomposites of silicon oxide in oxygen plasma-functionalized polylactic-co-glycolic acid (PLGA) resorbable membranes to favor osteoblast growth and proliferation during guided skull bone regeneration are clearly shown with rabbits used for experiments.

### Polymeric PLGA membrane elaboration

The membranes developed are resorbable organic scaffolds about 40 µm thick and functionalized on the nanometric scale with bioactive inorganic layers of SiO₂ of several tens of nanometers, produced in the Instituto de Ciencias de los Materiales (ICMSE), Seville.

The membranes are based on polylactic acid and/or polyglycolic polymers prepared according to a known method and are prepared by means of basic nanotechnological methodologies based on using cold plasmas and magnetron sputtering, to be subsequently functionalized.

### Polymeric membrane surface functionalization

Modifications were made on the surface of the membranes to promote greater osseointegration. These modifications include processes of coating with inorganic layers of titanium oxides (SiO₂) with potentially bioactive properties, i.e., surfaces that can form a direct chemical bond between the bone and the surface of the implant active fixing, equivalent to the concept of osseointegration used for the first time by Brånemark to describe the behavior of titanium dental implants in contact with bone.

To facilitate both the *in vivo* and *in vitro* degradation of PLGA membranes, they were subjected to treatment with oxygen plasma before depositing the inorganic layer on their surface. This plasma treatment (P-O₂) causes surface functionalization of the PLGA layer and increases its roughness due to the etching produced by the plasma. This treatment as a whole makes the membrane more sensitive to liquid culture media favoring its degradation. An improvement in the adherence between the nanometric inorganic layer and the PLGA membrane itself also occurs. These processes take place at temperatures close to room temperature and do not affect the integrity of the polymeric membrane, nevertheless, in addition to these samples, samples in the preparation of which treatment with P-O₂ has not been performed have been tested.

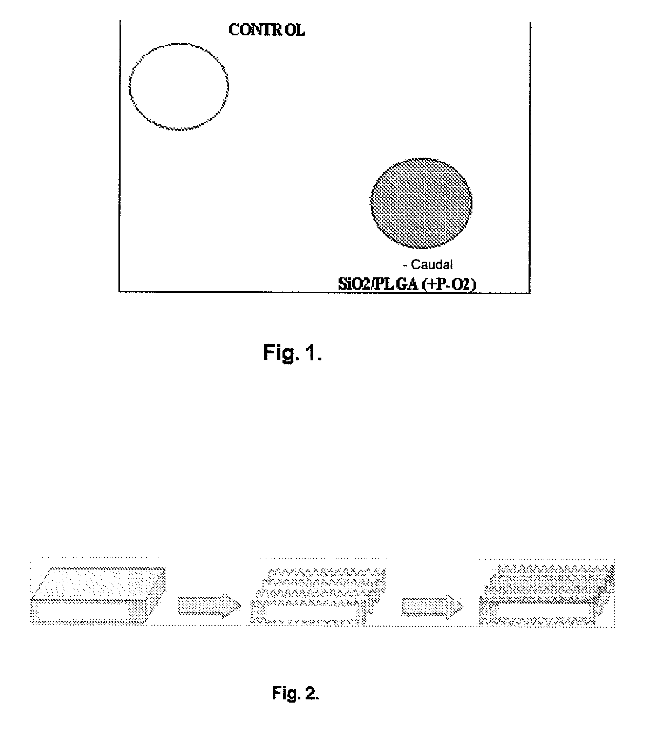

Figure 2 shows a diagram of this group of processes. Said Figure 2 shows how in a first step, treatment with oxygen plasma causes PLGA membrane surface activation increasing its roughness. The deposition of the bioactive nanometric inorganic layer takes place in a second step, the membrane then being ready for use.
*SiO₂*/*PLGA (*+*P-O₂)*
*- SIMPLE PLGA (CONTROL)*

### Specimens for animal experimentation

Surgical interventions were carried out in the Centro de Cirugía Mínimamente Invasiva Jesus Usón (CCMI, Caceres, Spain). Four white New Zealand rabbits used for experiments having identical characteristics were used for this study; (age: 6 months, weight: 3.5-4 kg), with a daily diet of the Harlan Teklad Lab Animal Diets (2030)-type rabbit maintenance diet.

### Animal experimentation method

The surgerical method used was GBG. It was started with immobilizing the animals with vital sign control. The following was used for the anesthesia process: intravenous induction with 0.25 vmg/kg midazolan and 5 mg/kg propofol, maintenance with aspirated 2.8% sevoflurane inhalation gas and analgesia of 1.5 mg/kg ketorolac and 3 mg/kg tramadol. Once the rabbits were sedated and prepared, an incision was made between the bases of the ears, communicating it with an incision in the midline of the tegmentum from the preceding incision to the front (about 5 cm) with a no. 15 scalpel blade. The epithelial, connective and muscle layers were then moved with a Pritchard periosteotome, controlling bleeding with compression and aspiration, exposing the outer surface of the skull and washing the area with a saline solution. Once the triangular flaps were peeled off, they were separated by means of a 2/0 suture, hanging two mosquito forceps from same to keep the field of intervention clear. Two structures, i.e., posterior bone crest and an outlet of an anterior nerve, were identified. Between both structures, three bone defects each separated by 3 mm were made on respective sides of the midline with a trephine mounted on a micromotor for implants with saline irrigation. The trephine used is a Helmut Zepf trephine with reference 08.910.13 having an internal diameter of 5 mm, teeth of 2.35 mm and length of 30 mm. The defect will be marked with a depth of 1 mm, and at 2000 rpm with said part. The bone will be removed by using piezosurgery and by controlling the depth at 2 mm by means of a periodontal probe.

Once the defects were made, they were covered with different PLGA membranes, as shown in Figure 1, placed according to the assigned group, following the organization below:

The membranes were fixed with a tissue adhesive (TissuCol®) placed at the bony rim adjacent to the defects to better fix the membrane, a suitable adhesion and low mobility when moving the flap to its initial position being verified. Suture was then made in three planes, i.e., a 4/0 suture in the periosteum plane, a 4/0 suture in the subepidermal plane, and a 2/0 suture on the skin, always using resorbable material. Simple stitches made as close as possible to the edges were used and the wound was cleaned. The approximate time of the operation was 1 hour per specimen.

After the surgery, 0.05 mg/kg buprenorphine and 1 ml/12.5 kg/wv carprofen anti-inflammatory analgesia was administered to the animals with monitored scheduled rest.

All the specimens were sacrificed using an overdose of potassium chloride in dissolution intravenously one month after the operation, and the samples were obtained from the tegmentum of each specimen, cut in the anatomical sagittal plane, and the parts were individually cut and labeled after separating the brain mass and washing with physiological serum. For laboratory manipulation, the samples were fixed with 70% alcohol for delivery to the histology laboratory.

### Sample processing

The histological processing of the tegmentum samples, once sacrificed, was carried out in the Facultad de Cirugia Dental, EA 2496 (Paris Descartes University), following the protocol below:
1. The first step will be an x-ray study (R-X) of the blocks for observing bone density and location of the defects in each group.
2. Inclusion in Merck's methyl methacrylate (MMA) (in blocks).
3. Carving the MMA blocks into a half-moon shape, in horizontal and following the plane perpendicular to the sagittal axis, in G-Brot 95370, polishing same in a G-Brot 95370 disc polisher.
4. Histological sections 5 µ thick made with a Jung Polycute microtome, at the level of the middle area of the bone defect (to minimize thickness variance between samples), in a series of 10 on glass holders pretreated with albumin and fixed with 80% alcohol.
5. Stainings:
   - Toluidine Blue
   - Von Kossa
6. Enzymology:
   - Acid phosphatase (TRAP)
   - Alkaline phosphatase (ALP)
7. Fluorescence:
   - Calcein

### Study of the percentage of neoformed bone

Merck's Toluidine Blue (BT) histological staining was used to compare the percentage of neoformed bone with respect to the original bone after defect regeneration. This staining is a common routine technique for a quick contrast analysis in a tissue. Furthermore, Toluidine Blue behaves like an orthochromatic dye; (blue color), or metachromatic dye (violet-red color), depending on the pH and the chemical nature of the stained substance, metachromatically staining structures rich in sulfated proteoglycans, such as heparan sulfate, present for example in young cartilage (chondroblasts and immature matrix) and in the granules of mast cells. Therefore, the osteoid tissue (new bone tissue with a higher percentage of type I collagenase matrix) and mature bone with a higher proportion of mineral, will be observed with a different blue tone since they have different pH. A 1% Toluidine Blue solution at a pH of 3.6 adjusted with HCl was used for this study. The time of exposing the samples to the dye was 10 minutes at room temperature, rinsing with distilled water and air drying.

To evaluate the resorptive activity of the neoformed bone tissue, the evidence of tartrate-resistant acid phosphatase (TRAP) enzymology technique was used, the TRAP being an acid hydrolase of osteoclasts and preosteoclasts released to the surrounding medium by these cells during bone resorption or reabsorption to facilitate bone dissolution, and it is linked to the degree of bone tissue vitality. To that end, the sections of the samples were incubated (in conditions of darkness) in Sigma naphthol ASTR phosphate previously dissolved in NN dimethyl formamide, to which 0.1 M acetate buffer, Sigma sodium tartrate and Fast Network TR Aldrich were added, adjusting the pH to 5.2 and filtering the final solution. The sections were incubated for 1 hour at 37°C. The positive result is reddish aggregates in the inside surrounding the osteoclasts, which allows quantifying the number thereof and the resorptive surface of the analyzed tissue.

### Apposition/resorption balance study

To evaluate the osteosynthetic activity of the tissue, the evidence of alkaline phosphatase (ALP) of osteoblasts and preosteoblasts technique was used, the ALP being a hydrolase enzyme marking osteosynthetic differentiation, responsible for forming type I collagen and calcified nodules. For the study, the sections were preincubated for 10 minutes in 0.1 % tris-triton, subsequently and after washing in 0.1 M pH 9 tris, they were incubated for 30 minutes (in darkness) and at 37°C in Sigma naphthol ASTR phosphate coupled to Sigma fast blue RR Salt (which develops the color reaction), with an adjusted pH of 9. A weak 1% Toluidine Blue stain diluted to 10% in distilled water was also added thereto to provide contrast to the samples. The positive result is violet colored cells. This staining allows defining the dimensions of the osteogenic band of the sample.

### Mineralization study

To illustrate the differences of mineralized bone tissue calcification between the different samples, the Sigma Von Kossa (VK) silver nitrate staining technique was used, this method corresponding to a method of replacing Ca with Ag. Silver nitrate only binds to the anionic part of the calcium salts (phosphates or carbonates), a yellow colored compound which turns black when exposed to the sun or to light due to silver reduction.

To measure the bone apposition rate and the bands of neoformed bone after the operation, the method of calcein-demeclocycline (fluorescent markers emitting at different wavelength, administered intravenously, signaling anabolic processes of bone tissue fixing) was used. The histomorphometric analysis of the presence of the marker and the distance between the signals is an effective method for determining and quantifying the bone growth and functional adaptation mechanisms. They offer a view of the neoformed bone tissue showing two differentiation lines corresponding to bone regenerated 1 day and 8 days before sacrificing the specimens. Calcein at 2% NaHCO₃ in serum (1 g SIGMA C0875) and demeclocycline hydrochloride (5 g SIGMA A6140).

### Morphometric study

The Zeis Axioscop 2 plus microscope with 4x, 10x and 20x objectives was used to view the bone effects. The Sony 3CCD (DSP) camera together with Smart Matrox Intelicam 8.0 software were used to obtain the images. To quantify the newly regenerated tissue and to compare same with the original tissue, the study areas were analyzed from distal (external) to proximal (internal) by means of ROI color analysis by regions with the Fiji-Image J software.

### Statistical analysis

A non-parametric Kruskal-Wallis analysis for n=4 was used for the statistical assessment of the obtained results, and U-Mann Whitney analysis was used for studying the degrees of individual significance between groups by means of Statview F-4.5 software (MAC).

### Results

### Light microscopy:

Taking average values ± S.D. of the results obtained in the study, after analysis with Toluidine Blue (BT), the following results were obtained:
- The percentages of neoformed bones measured after 1 month of rest following the operation show (39.7% and S.D. = 3.53) in SiO₂+P-O₂-functionalized membranes with respect to (30.57% and S.D. = 1.96) in the membranes of the control group.
- The digital captures obtained after silver nitrate staining (VK) illustrate the calcium phosphate formations during bone defect regeneration and confirms a greater density of mineral calcium in the SiO₂+P-O₂/PLGA type membranes with respect to simple PLGA control with insufficient calcification. The membranes offering less difference with respect to the level of neoformed bone are also the silicon oxide-functionalized membranes with a result of 2.9 with respect to 12 of the control group.
- Calcein analyses 8 days and 1 day before sacrificing the specimens show a mean result of 0.065 mm/day +/- 0.015 S.D. in the SiO₂+P-O₂ membranes with respect to 0.032 +/- 0.005 S.D. in the membranes of the control group with PLGA alone.
- The images obtained after enzymology analyses with TRAP and ALP show a normal osteoblastic and osteoclastic activity, suggesting an increase in activity in those SiO₂+P-O₂-functionalized membranes with 13.58 Oc/mm and 9.21 in the control group, demonstrating a normal apposition level and tissue vitality of the studied samples.
- Likewise, the membranes offering less difference with respect to the bone resorption level are also the ones functionalized with silicon oxide with a result of 7.9 with respect to a difference of 12.3 in the of the control group.

**Table 1. Percentage of bone after one month of regeneration**

| Neoformed bone (%) | R1 | R2 | R3 | R139 |
|---|---|---|---|---|
| SiO₂/PLGA + (P-O₂) | 42.3 | 33.6 | 48.5 | 34.4 |
| Control (PLGA) | 32 | 38.6 | 36 | 26 |
| Original Bone (O.B.) | 39.8 | 45.4 | | |

**Table 2. Differences between the percentages of bone of each study group with respect to the original**

| % Oss | Control (PLGA) | SiO₂/PLGA + PO₂ | O.B. |
|---|---|---|---|
| Mean | 30.57 | 39.7 | 42.6 |
| Standard deviation (S.D.) | 1.96 | 3.53 | 3.95 |
| Difference (O.B. - N.B.) | 12.03 | 2.9 | |
| | | | |

| Statistical significance (% OS) | | | |
|---|---|---|---|
| KW | | H = 6.441 | |
| p = | | 0.039* | |

**Table 3. Number of osteoclasts per millimeter after one month of regeneration**

| Bone resorption | R1 | R2 | R3 | R139 |
|---|---|---|---|---|
| SiO₂/PLGA + (P-O₂) | 12.3 | 15.8 | 12.2 | 14 |
| Control (PLGA) | 10.6 | 7.3 | 10.4 | 11.9 |

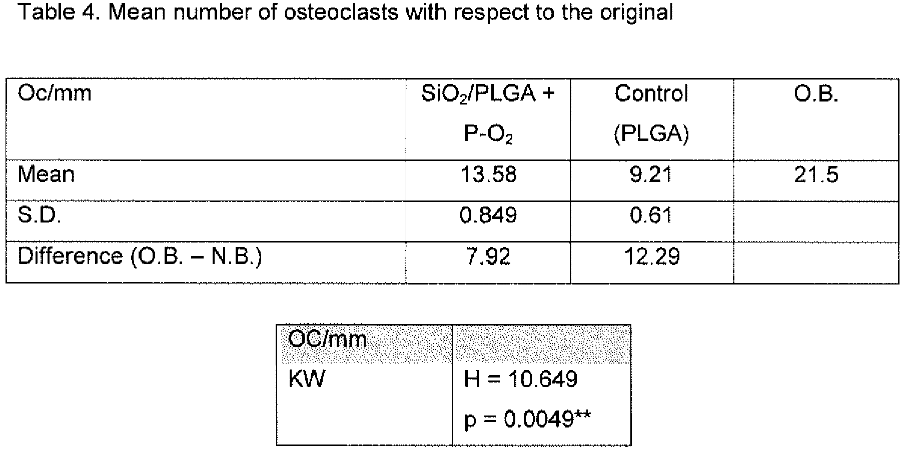

**Table 5. Calcein study: level of neoformed bone per day with respect to control**

| Calcein (mm/day) | Control | SiO₂ + (P-O₂) |
|---|---|---|
| 1 | 0.032 | 0.071 |
| 2 | 0.039 | 0.090 |
| 3 | 0.029 | 0.071 |
| 4 | 0.025 | 0.078 |
| 5 | 0.027 | 0.039 |
| 6 | 0.029 | 0.071 |
| 7 | 0.036 | 0.054 |
| 8 | 0.036 | 0.045 |
| 9 | 0.039 | 0.064 |
| 10 | 0.029 | 0.071 |
| Mean (mm/day) | 0.032 | 0.065 |
| S.D. +/- | 0.005 | 0.015 |

### Example 2. In vivo comparative evaluation of the nanocomposites of titanium oxide (TiO₂) in oxygen plasma-functionalized PLGA membranes by means of GBG

This is a model similar to the foregoing, with the same materials and methods, this time using titanium oxide (TiO₂)

The membranes tested *in vivo* for comparison were:
- TiO₂/PLGA (+P-O₂).
- SIMPLE PLGA (control).

### Results

### Light microscopy:

Taking average values ± S.D. of the results obtained in the study, after analysis with Toluidine Blue (BT), the following results were obtained:
- The percentages of neoformed bones measured after 1 month of rest following the operation show (45.67% and S.D. = 6.07) in SiO₂+P-O₂-functionalized membranes with respect to (30.57% and S.D. = 1.96) in control membranes.
- The digital captures obtained after silver nitrate staining (VK) illustrate the calcium phosphate formations during bone defect regeneration and confirms a greater density of mineral calcium in the TiO₂+P-O₂/PLGA type membranes with respect to the PLGA control group with minimum calcification. The membranes offering less difference with respect to the level of neoformed bone are also the titanium-functionalized membranes with a result of (-3.5), with respect to 12 of the control.
- Calcein analyses 8 days and 1 day before sacrificing the specimens show a mean result of 0.061 +/- 0.017 S.D. in the titanium membranes and 0.032 +/-0.005 S.D. in control membranes with PLGA alone.
- The images obtained after enzymology analyses with TRAP and ALP show a normal osteoblastic and osteoclastic activity, suggesting an increase in activity in those TiO₂+P-O₂-functionalized functionalized with 15.13 Oc/mm with respect to 9.21 Oc/mm in the membranes of the control group, demonstrating a normal apposition level and tissue vitality of the studied samples.

Likewise, the membranes offering less difference with respect to the bone resorption level are also the ones functionalized with titanium with a result of 6.4 with respect to 12.3 in the membranes of the control group.

**Table 6. Percentage of bone after one month of regeneration**

| Neoformed bone (%) | R4 | R5 | R132 | R145 |
|---|---|---|---|---|
| TiO₂/PLGA + (P-O₂) | 37.5 | 57 | 33.1 | 55 |
| Control (PLGA) | 24.4 | 33.3 | 23.2 | 31.08 |
| Original Bone (O.B.) | 39.98 | 45.4 | | |

**Table 7. Differences between percentages of bone of each study group with respect to the original**

| % Oss | Control (PLGA) | TiO₂/PLGA + P-O₂ | O.B. |
|---|---|---|---|
| Mean | 30.57 | 45.67 | 42.6 |
| S.D. | 1.96 | 6.07 | 3.95 |
| Difference (O.B. - N.B.) | 12.032 | -3.05 | |
| | | | |

| Statistical significance (% OS) | | | |
|---|---|---|---|
| KW | | H = 6.441 | |
| | | p = 0.039* | |

**Table 8. Number of osteoclasts per millimeter after one month of regeneration**

| Bone resorption | R4 | R5 | R132 | R145 |
|---|---|---|---|---|
| TiO₂/PLGA + (P-O₂) | 17.2 | 11.2 | 18.4 | 13.7 |
| Control (PLGA) | 8.8 | 9.4 | 6.8 | 8.5 |

**Table 9. Mean number of osteoclasts with respect to the original**

| | | | | |
|---|---|---|---|---|
| Bone resorption | R4 | R5 | R132 | R145 |
| TiO₂/PLGA + (P-O₂) | 17.2 | 11.2 | 18.4 | 13.7 |
| Control (PLGA) | 8.8 | 9.4 | 6.8 | 8.5 |
| | | | | |

| OC/mm | | | | |
|---|---|---|---|---|
| KW | H = 10.649 | | | |
| | p = 0.0049** | | | |

**Table 10. Calcein study. Level of neoformed bone per day with respect to control**

| Calcein (mm/day) | Control | TiO₂ + (P-O₂) |
|---|---|---|
| 1 | 0.032 | 0.068 |
| 2 | 0.039 | 0.090 |
| 3 | 0.029 | 0.054 |
| 4 | 0.025 | 0.036 |
| 5 | 0.027 | 0.082 |
| 6 | 0.029 | 0.060 |
| 7 | 0.036 | 0.045 |
| 8 | 0.036 | 0.060 |
| 9 | 0.039 | 0.075 |
| 10 | 0.029 | 0.043 |
| Mean (mm/day) | 0.032 | 0.061 |
| S.D. +/- | 0.005 | 0.017 |

### Example 3. In vivo comparative model of the nanocomposites of TiO₂ and SiO₂ with respect to treatment with O₉ plasma, on PLGA membranes in in vivo guided bone regeneration in rabbits

This is a comparative model of the two preceding models with a similar process and materials and methods.

The membranes tested for comparison were:
- PLGA,
- P-O₂/PLGA
- TiO₂/PLGA
- TiO₂+P-O₂/PLGA
- SiO₂/PLGA
- SiO₂+P-O₂/PLGA

### Results

Light microscopy:

Taking average values ± S.D. of the results obtained in the study, after analysis with Toluidine Blue (BT), the following results were obtained:
- The percentages of neoformed bones after 1 month of rest following the operation, and the means of the differences with respect to the percentage of original bone which have been obtained demonstrate that SiO₂+P-O₂-functionalized membranes (41.5% and D = 9.5) followed by SiO₂-functionalized membranes (42.4% and D = 11.53) have a higher percentage of neoformed bone. The TiO₂-functionalized membranes (32.83% and D = 10,47) are those following them in those parameters.
- In the case of the samples processed after 2 months, the membranes with a higher percentage of regenerated bone and less difference with the original are those having functionalizations of the type: SiO₂+P-O₂ (59.07% and D = -8.07) regenerating more bone than the existing original one. The membranes with TiO₂ (41.67 and D = 1.76) come in second as regards bone regenerating potential.
- The digital captures obtained after silver nitrate staining (VK) illustrate the calcium phosphate formations during bone defect regeneration and confirms the differences between the studied membranes, proving a greater density of mineral calcium in one-month old membranes with SiO₂+P-O₂/PLGA, followed by TiO₂/PLGA and TiO₂+P-O₂/PLGA with respect to the PLGA control with minimum calcification. In two-month old samples, after the synthetic-resorptive balance, the most outstanding membrane with respect to the control is SiO₂+P-O₂/PLGA membranes.
- The images obtained after enzymology analyses with TRAP and ALP show a normal osteoblastic and osteoclastic activity, suggesting an increase in activity in those treated with plasma and functionalized with SiO₂, and demonstrating a normal tissue vitality of the studied samples.

**Table 11: Percentage of bone (percentage of neoformed bone with respect to the original), original and neoformed bone, mean differences (differences between percentages of bone between the first month and the second month of regeneration with respect to the original), mineralized bone length, resorption 1 (resorptive length), resorption 2 (percentage of bone resorption), osteoclasts 1 (mean count of the no. of total osteoclasts), osteoclasts 2 (number of osteoclasts per millimeter of perimeter mineralized). (*) Samples R1 1037-5; (1), and the rest of the samples labeled with (-); (2) in the tables, are not present due to reasons relating to STOCK (1), and to defects as a result of deterioration during the inclusion or manipulation thereof (2), respectively.**

| | ANIMAL CODE | CONTROL PLGA (6) | PLGA/SIO₂ (1) | PLGA/TIO₂ + (P-O₂) (2) | PLGA/TIO₂ (3) | PLGA + (P-O₂) (4) | PLGA/SIO₂ + (P-O₂) (5) | CONTROL (NO MEMBRANE) |
|---|---|---|---|---|---|---|---|---|
| Percentage of bone | MEAN (D) 1m +/- S.D. | 39.77 +/- 8.52% | 42.40 +/- 16.83% | 41.27 +/- 14.56% | 32.83 +/- 19.22% | 34.77 +/-10.39& | 41.05 +/- 14.04% | 8% |
| | MEAN (O.B.) +/- S.D. | 55.63 +/- 24.99% | 53.93 +/- 17.59% | 69.13 +/- 10.19% | 43.43 +/- 12.47% | 63.73 +/-11.37% | 51.00 +/- 24.93% | 23.40% |
| | MEAN (D) 2m +/- S.D. | 38.2 +/- 22.67% | 41.8 +/- 10.38% | 39.5 +/- 8.94% | 41.67 +/- 4.80% | 50.27 +/- 5.35% | 59.07 +/- 12.15% | |
| Original and neoformed bone | MEAN (D) 1m | 39.77% | 42.4% | 41.27% | 32.83% | 34.77% | 41.5% | 8% |
| | MEAN (2m) | 38.2% | 41.8% | 39.5% | 41.67% | 50.27% | 59.07% | |
| | MEAN (O.B.) 1m | 55.63% | 53.93% | 69.13% | 43.43% | 63.73% | 51% | 23.4% |
| | DIFFERENCE (1m) | 15.86% | 11.53% | 27.86% | 10.47% | 28.96% | 9.5% | 15.4% |
| | | | | | | | | |
| Mean differences | DIFFERENCE (2m) | 17.40% | 12.13% | 29.63% | 1.76% | 13.46% | -8.07% | |
| | DIFFERENCES (1-2m) | 1.57% | 0.80% | 1.77% | -8.84% | -15.50% | -18.02% | |
| Mineralized bone length | MEAN (LTM) 1m | 17.32 +/- 3.09mm | 24.31 +/- 5.06mm | 16.66 +/- 2.74mm | 16.81 +/- 5.19mm | 17.32 +/- 4.42mm | 11.18 +/- 7.26mm | |
| | 1/x 1m | 0.058 mm | 0.041 mm | 0.060 mm | 0.059 mm | 0.058 mm | 0.107 mm | |
| | MEAN (LTM) 2m | 13.89 +/- 0.98mm | 15.29 +/- 0.81 mm | 16.74 +/- 3.68 mm | 14.82 +/- 2.33 mm | 17.49 +/- 4.75 mm | 14.73 +/- 2.33 mm | |
| | 1/x 1m | 0.072 mm | 0.065 mm | 0.060 mm | 0.067 mm | 0.057 mm | 0.068 mm | |
| Resorption 1 | MEAN (1m) | 0.66 +/-0.12 mm | 0.65 +/-0.09 mm | 0.78 +/-0.22 mm | 0.64 +/-0.36 mm | 0.54 +/-0.17 mm | 0.35 +/-0.19 mm | |
| | MEAN (2m) | 0.82 +/-0.37 mm | 0.95+/- 0.48 mm | 0.95 +/-0.21 mm | 0.57 +/-0.12 mm | 1.49+/- 0.95 mm | 0.68 +/-0.40 mm | |
| Resorption 2 | MEAN (1m) | 3.64 +/-0.59% | 4.20 +/-0.28% | 5.15 +/-1.94% | 3.59 +/-0.88% | 3.34 +/-1.48% | 3.32 0.70% | |
| | MEAN (2m) | 5.50 +/-1.73% | 6.47+/- 3.51% | 5.83+/- 0.10% | 4.45 +/-1.90% | 9.76 +/-8.27% | 6.70 +/-2.64% | |
| Osteoclasts 1 | MEAN (1m) | 18.5 +/- 2.50 | 18 +/- 1.0 | 24.5 +/- 4.58 | 19.5 +/-12.49 | 16+/-7.09 | 8.25 +/-3.89 | |
| | MEAN (2m) | 25.5 +/-13.44 | 30.5 +/- 12.68 | 32.75 +/-5.30 | 20.33 +/-6.03 | 44 +/- 31.11 | 29 +/-17.84 | |
| Osteoclasts 2 | MEAN (1m) | 0.91 +/- 0.46 | 1.20 +/- 0.19 | 1.59 +/- 0.50 | 1.06 +/-0.35 | 0.98 +/- 0.55 | 0.83 +/- 0.24 | |
| | MEAN (2m) | 1.79 +/- 0.87 | 2.10 +/- 0.98 | 1.99 +/- 0.12 | 1.58 +/-0.83 | 2.87 +/- 2.56 | 2.02 +/-1.35 | |

## Claims

1. A membrane comprising a biodegradable polymer obtainable by a method which comprises exposing at least one face of a polymeric film to the effect of a plasma.

2. The membrane according to the preceding claim, where the plasma is made up of oxygen, argon, CO₂, N₂O or any of the mixtures thereof.

3. The membrane according to any of claims 1-2, where the polymer is selected from polylactic acid, polyglycolic acid or any of the mixtures thereof.

4. The membrane according to any of claims 1-3 obtainable by a method which further comprises depositing one or more nanometric layers of active oxides on one or both of the faces of the polymeric film.

5. The membrane according to the preceding claim, where the active oxides are selected from the list consisting of SiO₂, TiO₂, ZnO, hydroxyapatite or derivatives, or other oxides that are neither toxic nor have side effects and favor osteoblast growth, or any of the combinations thereof.

6. The membrane according to any of claims 4-5, where the deposition of nanometric layers of active oxides is carried out by means of dry techniques.

7. The membrane according to the preceding claim, where the dry techniques are selected from the list consisting of physical vapor deposition (PVD), sputtering, plasma enhanced chemical vapor deposition (PECVD), pulsed laser deposition (PLD), any similar technique or any of the combinations thereof.

8. The membrane according to any of claims 4-7, where the nanometric layer or layers of active oxides have a thickness ranging between 10 nm and 1 micron.

9. The membrane according to any of claims 4-8, where the nanometric layer or layers of active oxides are deposited on the face of the film or membrane not treated with plasma.

10. The membrane according to any of claims 1-9, where the time of exposing the face of the polymeric film to the effect of the plasma ranges between 10 and 40 minutes.

11. An implant comprising a membrane according to any of claims 1-10.

12. A monolithic or articular implant according to the preceding claim which is selected from sutures, staples, prostheses, screws or plates.

13. Use of the membrane according to any of claims 1-10 in the elaboration of a medicinal product.

14. Use of the membrane according to any of claims 1-10 in the elaboration of a medicinal product for tissue regeneration.

15. Use of the membrane according to any of claims 1-10 in the elaboration of a medicinal product for bone tissue regeneration.

16. Use of the membrane according to any of claims 1-10 in the elaboration of a medicinal product for inducing bone tissue neoformation.

17. Use of the membrane according to any of claims 1-10 in the elaboration of a medicinal product for repairing parts of skeletal muscle tissue.

18. A method for obtaining the membrane according to any of claims 1-10, which comprises:
(a) producing a resorbable film from biodegradable polymer or polymers;
(b) activating one or both of the faces of the film according to (a) by means of oxygen plasma or plasma made up of a mixture of gases,
(c) depositing a first layer of active material on one or both of the faces of the activated film of (b) using dry techniques that do not alter the structural integrity of the polymeric base material.

19. The method according to the preceding claim, where the active material of step (c) is inorganic.

20. The method according to any of claims 18 and 19, which further comprises:
(d) incorporating a second or more layers in multilayer form on the first layer to achieve multifunctional membranes suitable for various clinical processes.

21. The method according to any of claims 18-20, which further comprises:
(e) depositing nanometric layers having a mixed composition by means of combining one or more simultaneous or successive deposition processes.
